(19) Europäisches Patentamt
European Patent Office
Office européen des brevets

(11) EP 2 307 486 B2

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**23.10.2019 Patentblatt 2019/43**

(45) Hinweis auf die Patenterteilung:
**12.10.2016 Patentblatt 2016/41**

(21) Anmeldenummer: **09781227.5**

(22) Anmeldetag: **29.07.2009**

(51) Int Cl.:
***C08J 3/075*** *(2006.01)* ***A61L 15/42*** *(2006.01)*

(86) Internationale Anmeldenummer:
**PCT/EP2009/059793**

(87) Internationale Veröffentlichungsnummer:
**WO 2010/012762 (04.02.2010 Gazette 2010/05)**

(54) **FARBSTABILER SUPERABSORBER**

COLOUR-STABLE SUPERABSORBENT

SUPERABSORBANTS À STABILITÉ DE COULEUR

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **31.07.2008 EP 08161572**

(43) Veröffentlichungstag der Anmeldung:
**13.04.2011 Patentblatt 2011/15**

(73) Patentinhaber: **BASF SE**
**67056 Ludwigshafen am Rhein (DE)**

(72) Erfinder:
- **RIEGEL, Ulrich**
**66849 Landstuhl (DE)**
- **DANIEL, Thomas**
**67165 Waldsee (DE)**
- **HERFERT, Norbert**
**63674 Altenstadt (DE)**
- **ELLIOTT, Mark**
**67063 Ludwigshafen (DE)**

(74) Vertreter: **BASF IP Association**
**BASF SE**
**G-FLP-C006**
**67056 Ludwigshafen (DE)**

(56) Entgegenhaltungen:
**WO-A-2004/084962** **WO-A1-2009/011717**
**WO-A1-2009/060062**

EP 2 307 486 B2

**Beschreibung**

Die vorliegende Erfindung betrifft einen farbstabilen Superabsorber, ein Verfahren zu seiner Herstellung sowie seine Verwendung und ihn enthaltende Hygieneartikel. Unter einem farbstabilen Superabsorber ist ein Superabsorber zu verstehen, der sich bei Lagerung unter erhöhter Temperatur und Luftfeuchtigkeit nicht oder nur in vergleichsweise geringem Maße verfärbt.

Superabsorber sind bekannt. Für derartige Materialien sind auch Bezeichnungen wie "hochquellfähiges Polymer" "Hydrogel" (oft auch für die trockene Form verwendet), "Hydrogel bildendes Polymer", "Wasser absorbierendes Polymer", "absorbierendes gelbildendes Material", "quellfähiges Harz", "wasserabsorbierendes Harz" oder ähnliche gebräuchlich. Es handelt sich dabei um vernetzte hydrophile Polymere, insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate, wobei wasserabsorbierende Polymere auf Basis teilneutralisierter Acrylsäure am weitesten verbreitet sind. Die wesentlichen Eigenschaften von Superabsorbern sind ihre Fähigkeiten, ein Vielfaches ihres Eigengewichts an wässrigen Flüssigkeiten zu absorbieren und die Flüssigkeit auch unter gewissem Druck nicht wieder abzugeben. Der Superabsorber, der in Form eines trockenen Pulvers eingesetzt wird, wandelt sich bei Flüssigkeitsaufnahme in ein Gel, bei der üblichen Wasseraufnahme entsprechend in ein Hydrogel um. Die Vernetzung ist für synthetische Superabsorber wesentlich und ein wichtiger Unterschied zu üblichen reinen Verdickern, da sie zur Unlöslichkeit der Polymeren in Wasser führt. Lösliche Substanzen wären als Superabsorber nicht brauchbar. Das mit weitem Abstand wichtigste Einsatzgebiet von Superabsorbern ist das Absorbieren von Körperflüssigkeiten. Superabsorber werden beispielsweise in Windeln für Kleinkinder, Inkontinenzprodukten für Erwachsene oder Damenhygieneprodukten verwendet. Andere Anwendungsgebiete sind beispielsweise die als wasserzurückhaltende Mittel im landwirtschaftlichen Gartenbau, als Wasserspeicher zum Schutz vor Feuer, zur Flüssigkeitsabsorption in Lebensmittelverpackungen oder ganz allgemein zur Absorption von Feuchtigkeit.

Superabsorber können ein mehrfaches ihres Eigengewichts an Wasser absorbieren und unter gewissem Druck zurückhalten. Im Allgemeinen weist ein derartiger Superabsorber eine CRC ("Centrifuge Retention Capacity", Meßmethode siehe unten) von mindestens 5 g/g, vorzugsweise mindestens 10 g/g und in besonders bevorzugter Form mindestens 15 g/g auf. Ein "Superabsorber" kann auch ein Gemisch stofflich verschiedener einzelner Superabsorber sein oder ein Gemisch von Komponenten, die erst im Zusammenwirken superabsorbierende Eigenschaften zeigen, es kommt hier weniger auf die stoffliche Zusammensetzung an als auf die superabsorbierenden Eigenschaften.

Wichtig für einen Superabsorber ist nicht nur seine Absorptionskapazität, sondern auch die Fähigkeit, Flüssigkeit unter Druck zurückzuhalten (Retention) sowie der Flüssigkeitstransport im gequollenen Zustand. Gequollenes Gel kann den Flüssigkeitstransport zu noch nicht gequollenem Superabsorber behindern bis verhindern ("gel blocking"). Gute Transporteigenschaften für Flüssigkeiten besitzen beispielsweise Hydrogele, die im gequollenen Zustand eine hohe Gelfestigkeit aufweisen. Gele mit nur geringer Gelfestigkeit sind unter einem angewendetem Druck (Körperdruck) deformierbar, verstopfen Poren in dem Superabsorber / Cellulosefaser-Saugkörper und verhindern dadurch eine weitere Flüssigkeitsaufnahme. Eine erhöhte Gelfestigkeit wird in aller Regel durch einen höheren Vernetzungsgrad erreicht, wodurch allerdings Absorptionskapazität des Produktes verringert wird. Eine elegante Methode zur Erhöhung der Gelfestigkeit stellt die Erhöhung des Vernetzungsgrads an der Oberfläche der Superabsorberpartikel gegenüber dem Inneren der Partikel dar. Dazu werden meist in einem Oberflächennachvernetzungsschritt getrocknete Superabsorberpartikel mit durchschnittlicher Vernetzungsdichte einer zusätzlichen Vernetzung in einer dünnen Oberflächenschicht ihrer Partikel unterworfen. Durch die Oberflächennachvernetzung steigt die Vernetzungsdichte in der Schale der Superabsorberpartikel, wodurch die Absorption unter Druckbelastung auf ein höheres Niveau gehoben wird. Während die Absorptionskapazität in der Oberflächenschicht der Superabsorberpartikel sinkt, weist ihr Kern durch das Vorhandensein beweglicher Polymerketten eine im Vergleich zur Schale verbesserte Absorptionskapazität auf, so dass durch den Schalenaufbau eine verbesserte Flüssigkeitsweiterleitung gewährleistet wird, ohne dass gel blocking auftritt. Es ist ebenfalls bekannt, insgesamt höher vernetzte Superabsorber zu erzeugen und den Vernetzungsgrad im Inneren der Partikel gegenüber einer äußeren Schale der Partikel nachträglich zu verringern.

Auch Verfahren zur Herstellung von Superabsorbern sind bekannt. Superabsorber auf Basis von Acrylsäure, die auf dem Markt am gängigsten sind, werden durch radikalische Polymerisation von Acrylsäure in Gegenwart eines Vernetzers (dem "Innenvernetzer") hergestellt, wobei die Acrylsäure vor, nach oder teils vor, teils nach der Polymerisation zu einem gewissen Grad neutralisiert wird, üblicherweise durch Zugabe von Alkali, meist einer wässrigen Natriumhydroxidlösung. Das so gewonnene Polymergel wird zerkleinert (je nach verwendetem Polymerisationsreaktor kann dies gleichzeitig mit der Polymerisation erfolgen) und getrocknet. Das so gewonnene trockene Pulver (das "Grundpolymer" oder "Basispolymer") wird üblicherweise an der Oberfläche der Partikel nachvernetzt, indem es mit weiteren Vernetzern wie etwa organischen Vernetzern oder mehrwertigen Kationen, beispielsweise Aluminium (meist als Aluminiumsulfat eingesetzt) oder beidem umgesetzt wird, um eine gegenüber dem Partikelinneren stärker vernetzte Oberflächenschicht zu erzeugen.

Ein bei Superabsorbern oft auftretendes Problem sind Verfärbungen, die beim Lagern unter höherer Temperatur oder höherer Luftfeuchtigkeit auftreten. Derartige Bedingungen treten oft bei Lagerung von Superabsorbern in tropischen oder subtropischen Ländern auf. Unter solchen Bedingungen neigen Superabsorber zum Vergilben, sie können sogar braune oder gar fast schwarze Färbung annehmen. Diese Verfärbung des eigentlich farblosen Superabsorberpulvers ist unansehnlich und unerwünscht, da sie insbesondere bei den erwünschten dünnen Hygieneprodukten sichtbar ist und Verbraucher unansehnliche Hygieneprodukte ablehnen. Die Ursache für die Verfärbung ist nicht vollständig geklärt, allerdings scheinen reaktive Verbindungen wie Restmonomere aus der Polymerisation, die Verwendung mancher Initiatoren, Verunreinigungen des Monomeren oder des Neutralisationsmittels, Oberflächennachvernetzer oder Stabilisatoren der verwendeten Monomere eine Rolle zu spielen.

Fredric L. Buchholz und Andrew T. Graham (Hrsg.) geben in: "Modern Superabsorbent Polymer Technology", J. Wiley & Sons, New York, U.S.A. / Wiley-VCH, Weinheim, Germany, 1997, ISBN 0-471-19411-5, einen zusammenfassenden Überblick über Superabsorber, ihre Eigenschaften und Verfahren zur Herstellung von Superabsorbern.

WO 2008/055856 A1 lehrt die Vermeidung von Verfärbungen eines Superabsorbers, die durch zu hohen Eisengehalt der Natronlauge hervorgerufen werden, die zur Teilneutralisation der Acrylsäure bei der Herstellung des Superabsorbers verwendet wird, durch Zusatz von Phosphorsäure oder Phosphatsalzen. JP 05/086 251 A lehrt die Verwendung von Phosphorsäurederivaten oder Salzen davon, insbesondere 1-Hydroxiethyliden-1,1-diphosphonsäure, Ethylendiamintetra(methylenphosphonsäure), Diethylentriaminpenta(methylenphosphonsäure) oder deren Alkali- oder Ammoniumsalze als Stabilisatoren von Superabsorbern gegen Verfärbung. WO 03/059 962 A1 oder die äquivalente Patentanmeldung US 2005/0085604 A1 offenbart die Verwendung von Metall-Chelatisierungsmitteln in irgendeinem Schritt der Superabsorberherstellung sowie die Hinzufügung eines Reduktions- oder Oxidationsmittels vor Trocknung des wasserhaltigen Polymerisats als Maßnahmen gegen Verfärbung. WO 03/014 172 A2 betrifft die Herstellung von Superabsorbern aus hochreiner Acrylsäure, die zur Vermeidung von Verfärbungen der Superabsorber insbesondere von Aldehyden befreit wurde. WO 00/55245 A1 lehrt die Stabilisierung von Superabsorbern gegen Verfärbung durch Behandlung mit einem anorganischen Reduktionsmittel und wahlweise einem Metallsalz. Das anorganische Reduktionsmittel ist typischerweise ein Hypophosphit, Phosphit, Bisulfit oder Sulfit. Das Metallsalz ist typischerweise ein farbloses (die Eigenschaft "farblos" wird oft auch einfach "weiß" genannt) Phosphat, Acetat oder Laktat, aber kein Halogenid. Nach der Lehre von WO 2006/058 682 werden Verfärbungen von Superabsorbern vermieden, wenn die Trocknung und die Nachvernetzungsreaktion in einer Atmosphäre durchgeführt werden, die im Wesentlichen frei von oxidierenden Gasen ist.

EP 505 163 A1 offenbart die Verwendung einer Kombination von oberflächenaktivem Stoff und einer sich an Doppelbindungen addierenden Verbindung wie beispielsweise unsubstitutierte oder substituierte Alkyl- oder Arylsulfinsäuren oder deren Salze zur Verringerung von Restmonomeren in Superabsorbern. EP 668 080 A2 sowie die Teilanmeldung EP 1570 869 A1 betreffen die Verwendung von organischen Säuren, einschließlich Sulfinsäuren, aber ausschließlich von Salzen organischer Säuren oder Sulfinsäuren, oder von Polyaminosäuren oder deren Salzen zur Verringerung restlichen Oberflächennachvernetzers, insbesondere von als Oberflächennachvernetzer verwendeten Epoxiverbindungen, nach der Oberflächennachvernetzung. Nach der Lehre von EP 386 897 A2, EP 441 975 A1 und EP 605 215 A1 lehren die Verwendung von Sulfiten, Hydrogensulfiten oder Thiosulfaten zur Verringerung von Restmonomeren aus der Polymerisation. EP 1 645 596 A1 lehrt die Stabilisierung von Superabsorbern gegen Verfärbung durch Zusatz eines anorganischen Salzes, eines Aminocarboxisäure-Chelatisierungsmittels und eines organischen Antioxidationsmittels. Als anorganisches Salz werden Sulfite, Bisulfite, Pyrosulfite, Dithionite, Trithionate, Tetrathionate, Thiosulfate oder Nitrite verwendet. EP 1 577 349 A1 lehrt die Verwendung dieser Salze zum selben Zweck, wobei jedoch der Gehalt des damit behandelten Superabsorbers an Eisen unter 1 Gew.-ppm gehalten wird. US 2009/0023848 A1 offenbart die Behandlung eines Superabsorbers mit einem Antioxidans, beispielsweise 2-Hydroxisulfinatoessigsäure, zum Zwecke der Stabilisierung gegen Verfärbungen.

EP 1 199 315 A2 lehrt die Verwendung eines Redoxinitiatorsystems zur Initiierung einer Polymerisationsreaktion, wobei das Redoxinitiatorsystem als reduzierenden Teil eine Sulfinsäure oder ein Sulfinat, insbesondere 2-Hydroxisulfinatoessigsäure oder ein Salz davon enthält. WO 99/18067 A1 offenbart bestimmte Hydroxi- oder Aminoalkyl- oder aryl-sulfinsäurederivate oder Gemische davon und ihre Verwendung als Reduktionsmittel, die keinen Formaldehyd abspalten. WO 2004/084 962 A1 betrifft die Verwendung dieser Sulfinsäurederivate als reduzierenden Teil eines Redoxinitiators zur Polymerisation teilneutralisierter Acrylsäure zur Superabsorbern. Initiatoren werden in geringer Menge verwendet, da zu große Initiatormengen zu unerwünscht kurzen Polymerketten und in Folge unerwünscht hohem Gehalt des Polymers an extrahierbaren Anteilen führen Die Bestandteile eines Redoxinitiators reagieren zudem im Zuge der Polymerisation ab, zeigen im fertigen Polymer keinen Effekt und sind im fertigen Polymer im Normalfall nicht mehr nachweisbar. Bei manchen Initiatoren können aber auch in unerwünschter Weise im Polymer verbleibende gefärbte Reaktionsprodukte entstehen. In der Regel genügen einige Routineversuche, geeignete Initiatoren zu finden.

Die ältere internationale Patentanmeldung mit Aktenzeichen PCT/EP2008/051009 lehrt den Zusatz eines basischen Salzes eines zweiwertigen Metallkations zu Superabsorbern, unter anderem, um die Stabilität gegen Verfärbung zu erhöhen. Die ältere internationale Patentanmeldung mit dem Aktenzeichen PCT/EP2008/051010 offenbart die Verwendung von Carbonsäuresalzen und/oder basischen Salzen dreiwertiger Metallkationen zum selben Zweck.

Es besteht weiterhin die Aufgabe, andere oder sogar besser gegen Verfärbung, insbesondere gegen Vergilbung oder Braunfärbung bei Lagerung unter erhöhter Temperatur und/oder erhöhter Luftfeuchtigkeit stabilisierte Superabsorber zu finden. Die Gebrauchseigenschaften des Superabsorbers, insbesondere seine Aufnahmefähigkeit für Flüssigkeit, auch unter Druck, sowie seine Fähigkeit zur Weiterleitung von Flüssigkeit, aber auch seine Rieselfähigkeit sollen dabei nicht oder zumindest nicht wesentlich beeinträchtigt werden. Weitere Aufgaben der Erfindung sind das Finden eines Verfahrens zur Herstellung eines derartigen Superabsorbers sowie von Verwendungen dieses Superabsorbers.

Gelöst wurde die Aufgabe durch einen Superabsorber, enthaltend eine Verbindung der Formel (I)

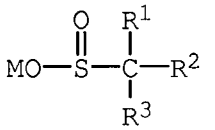

(I),

worin

M für ein Wasserstoffatom, ein Ammoniumion, ein einwertiges Metallion oder ein Äquivalent eines zweiwertigen Metallions der Gruppen 1, 2, 8, 9, 10, 12 oder 14 des Periodensystems der Elemente steht;

$R^1$ für OH oder $NR^4R^5$ steht, wobei $R^4$ und $R^5$ unabhängig voneinander für H oder $C_1$-$C_6$-Alkyl stehen;

$R^2$ für H oder eine Alkyl-, Alkenyl-, Cycloalkyl- oder Arylgruppe steht, wobei diese Gruppe wahlweise 1, 2 oder 3 Substituenten aufweist, die unabhängig voneinander ausgewählt sind unter $C_1$-$C_6$-Alkyl, OH, O-$C_1$-$C_6$-Alkyl, Halogen und $CF_3$; und

$R^3$ für COOM, $SO_3M$, $COR^4$,$CONR^4R^5$ oder $COOR^4$ steht, wobei M, $R^4$ und $R^5$ die oben angegebenen Bedeutungen besitzen oder, wenn $R^2$ für Aryl steht, das wahlweise wie oben angegeben substituiert ist, auch für H steht,

Salzen davon oder Gemischen solcher Verbindungen und/oder Salzen davon, der dadurch gekennzeichnet ist, dass er oberflächennachvernetzt ist, wobei die Nachvernetzungsreaktion bei einem Partialdruck oxidierender Gase von weniger als 140 mbar durchgeführt wurde,
sowie durch einen Superabsorber, enthaltend eine Verbindung der obigen Formel (I), Salzen davon oder Gemischen solcher Verbindungen und/oder Salzen davon, der dadurch gekennzeichnet ist, dass er weiterhin Phosphinat und/oder Phosphonat enthält.

Weiterhin wurden ein Verfahren zur Herstellung dieser Superabsorber gefunden, Verwendungen dieser Superabsorber sowie Hygieneartikel, die diese Superabsorber enthalten.

Die erfindungsgemäßen, die durch die Formel beschriebenen Sulfinsäurederivate enthaltenden Superabsorber zeigen überraschend gute Stabilität gegen Verfärbung, ohne dass ihre Gebrauchseigenschaften beeinträchtigt wären.

In obiger Formel steht Alkyl für geradkettige oder verzweigte Alkylgruppen, die vorzugsweise 1 - 6, insbesondere 1 - 4 Kohlenstoffatome aufweisen. Beispiele für Alkylgruppen sind Methyl, Ethyl, n-Propyl, Isopropyl, n-Butyl, t-Butyl, n-Hexyl etc. Entsprechendes gilt für die Alkylgruppen in O-Alkyl. Alkenyl steht für geradkettige oder verzweigte Alkenylgruppen, die vorzugsweise 3 - 8 Kohlenstoffatome, insbesondere 3 - 6 Kohlenstoffatome aufweisen. Eine bevorzugte Alkenylgruppe ist die Allylgruppe. Cycloalkyl steht insbesondere für $C_1$-$C_6$-Cycloalkyl, wobei Cyclopentyl und Cyclohexyl besonders bevorzugt sind. Aryl (auch in Aralkyl) steht vorzugsweise für Phenyl oder Naphthyl. Wenn der Arylrest für eine Phenylgruppe steht und substituiert ist, so weist er vorzugsweise zwei Substituenten auf. Diese sind insbesondere in 2- und/oder 4-Stellung vorhanden.

Halogen steht für F, Cl, Br und I, vorzugsweise für Cl und Br.

M steht vorzugsweise für ein Ammonium-, Alkalimetall- oder ein Äquivalent eines Erdalkalimetall- oder Zinkions. Geeignete Alkalimetallionen sind insbesondere Natrium- und Kaliumionen, geeignete Erdalkalimetallionen sind vor allem Magnesium-, Strontium- und Calciumionen.

$R^1$ steht vorzugsweise für eine Hydroxi- oder Aminogruppe. $R^2$ steht vorzugsweise für ein Wasserstoffatom oder eine Alkyl- oder Arylgruppe, die wie oben substituiert sein kann. Vorzugsweise weist sie einen oder zwei Hydroxi- und/oder Alkoxisubstituenten auf.

$R^3$ steht vorzugsweise entweder für COOM oder $COOR^4$ (M und $R^4$ besitzen die oben angegebenen Bedeutungen) oder, wenn $R^1$ für Aryl steht, das wie oben angegeben substituiert sein kann, auch für ein Wasserstoffatom.

In einer bevorzugten Ausführungsform enthält der Superabsorber Verbindungen der obigen Formel, worin M für ein Alkalimetallion oder ein Äquivalent eines Erdalkalimetall- oder Zinkions steht; $R^1$ für eine Hydroxi- oder Aminogruppe steht; $R^2$ für H oder Alkyl steht und $R^3$ für COOM oder $COOR^4$ steht, wobei, wenn $R^3$ für COOM steht, M in diesem COOM-Rest für H, ein Alkalimetallion oder ein Äquivalent eines Erdalkalimetallions steht und wenn $R^3$ für $COOR^4$ steht, $R^4$ für $C_1$-$C_6$-Alkyl steht.

In einer weiteren bevorzugten Ausführungsform enthält der Superabsorber Verbindungen der obigen Formel, worin M für ein Alkalimetallion oder ein Äquivalent eines Erdalkalimetall- oder Zinkions steht; $R^1$ für eine Hydroxi- oder Aminogruppe steht; $R^2$ für Aryl, das wahlweise wie oben angegeben substituiert ist, insbesondere für Hydroxiphenyl oder $C_1$-$C_4$-Alkoxyphenyl steht; und $R^3$ für ein Wasserstoffatom steht.

Die Gruppen 1 (H, Li, Na, K, Rb, Cs, Fr), 2 (Be, Mg, Ca, Sr, Ba, Ra), 8 (Fe, Ru, Os), 9 (Co, Rh, Ir), 10 (Ni, Pd, Pt), 12 (Zn, Cd, Hg) und 14 (C, Si, Ge, Sn, Pb) des Periodensystems der Elemente in der aktuellen Nummerierung der IUPAC (International Union of Pure and Applied Chemistry, 104 T.W. Alexander Drive, Building 19, Research Triangle Park, NC 27709, U.S.A., www.iupac.org), der für Nomenklatur im Bereich der Chemie zuständigen internationalen Organisation, entsprechen den Gruppen Ia, IIa, IIb, IVa und VIIIb in der von CAS (Chemical Abstracts Service, 2540 Olentangy River Road, Columbus, OH 43202, U.S.A., www.cas.org) verwendeten Nummerierung.

Die Sulfinsäurederivate der obigen Formel können in Reinform verwendet werden, wahlweise aber auch in dem aus der Herstellung solcher Verbindungen auf übliche Weise resultierenden Gemisch mit dem Sulfit des entsprechenden Metallions und der entsprechenden Sulfonsäure. Die Herstellung derartiger Sulfinsäurederivate der obigen Formel ist bekannt und beispielsweise in WO 99/18 067 A1 beschrieben. Sie sind auch gängige Handelswaren und beispielsweise in der Form von Gemischen aus dem Natriumsalz der 2-Hydroxy-2-sulfinatoessigsäure, dem Dinatriumsalz der 2-Hydroxy-2-sulfonatoessigsäure und Natriumbisulfit von L. Brüggemann KG (Salzstraße 131, 74076 Heilbronn, Deutschland, www.brueggemann.com) unter den Bezeichnungen BRÜGGOLIT® FF6M oder BRÜGGOLIT® FF7, alternativ BRUGGOLITE® FF6M oder BRUGGOLITE® FF7 erhältlich.

Bei der Herstellung der erfindungsgemäßen Superabsorber wird ein an sich bekannter Superabsorber mit einer Verbindung der Formel (I), einem Salz davon oder einer Mischung von Verbindungen der Formel (I) und/oder deren Salzen gemischt. Der Einfachheit halber ist im Folgenden stets nur von "Verbindung der Formel (I)" oder von "Sulfinsäure-Derivat" die Rede, dies soll jedoch Salze von derartigen Verbindungen und Gemische von derartigen Verbindungen und/oder ihren Salzen nicht ausschließen, sondern lediglich ihre ständige Mitnennung vermeiden.

Im Allgemeinen enthalten die erfindungsgemäßen Superabsorber mindestens 0,0001 Gew.-% Sulfinsäure-Derivat, vorzugsweise mindestens 0,001 Gew.-% und in besonders bevorzugter Form mindestens 0,025 Gew.-%, beispielsweise mindestens 0,05 Gew.-% oder mindestens 0,1 Gew.-% sowie im Allgemeinen höchstens 3 Gew.-%, in bevorzugter Form höchstens 2 Gew.-% und in besonders bevorzugter Form höchstens 0,5 Gew.-%, beispielsweise höchstens 0,35 Gew.-% oder 0,2 Gew.-%, jeweils auf das Gesamtgewicht des erfindungsgemäßen Superabsorbers bezogen.

Die Mischung von an sich bekannten Superabsorbern mit einer Verbindung der Formel (I) kann durch jedes bekannte Mischverfahren erfolgen. Die Verbindung der Formel (I) kann in Substanz, als Lösung oder als Suspension in einem Lösungs- oder Suspensionsmittel eingemischt werden, vorzugsweise wird sie aufgrund der leichteren homogenen Verteilung als Lösung oder Suspension eingemischt. Dabei wird nicht notwendigerweise eine einfach durch mechanische Maßnahmen trennbare physikalische Mischung von an sich bekanntem Superabsorber und Sulfinsäure-Derivat der Formel (I) erzeugt. Das Sulfinsäure-Derivat kann durchaus eine festere Verbindung mit dem Superabsorber eingehen, beispielsweise als vergleichsweise fest anhaftende Oberflächenschicht oder als fest an der Oberfläche der Superabsorberpartikel anhaftende Partikel des Sulfinsäure-Derivats. Die Einmischung des Sulfinsäure-Derivats in den an sich bekannten Superabsorber kann durchaus auch als "Beschichtung" verstanden und bezeichnet werden.

Falls der an sich bekannte Superabsorber mit einer Lösung oder Suspension des Sulfinsäure-Derivats vermischt wird, wird als Lösungs- oder Suspensionsmittel ein Lösungs- oder Suspensionsmittel verwendet, das sowohl mit dem Superabsorber wie auch mit dem Sulfinsäure-Derivat chemisch verträglich ist, also keine unerwünschten chemischen Reaktionen damit eingeht. Typischerweise wird Wasser oder ein organisches Lösungsmittel verwendet, beispielsweise ein Alkohol oder Polyol, oder Mischungen davon. Beispiele geeigneter Lösungs- oder Suspensionsmittel sind Wasser, I-sopropanol/Wasser, 1,3-Propandiol/Wasser und Propylenglykol/Wasser, wobei das Mischungsmassenverhältnis vorzugsweise von 20:80 bis 40:60 beträgt. Der Lösung oder Suspension kann ein Tensid zugesetzt werden.

Das Sulfinsäure-Derivat wird im Allgemeinen auf genau die gleiche Weise mit dem an sich bekannten Superabsorber vermischt wie die zur Oberflächennachvernetzung auf den Superabsorber aufgebrachte, einen Oberflächennachvernetzer enthaltende Lösung oder Suspension, wie weiter unten beschrieben. Das Sulfinsäure-Derivat kann als Bestandteil der zur Oberflächennachvernetzung aufgebrachten Lösung oder einer ihrer Komponenten auf einen (noch) nicht nachvernetzten Superabsorber (ein "Grundpolymer" oder "Basispolymer") aufgebracht, das Sulfinsäure-Derivat also der Lösung des Oberflächennachvernetzers oder einer ihrer Komponenten zugesetzt werden. Der mit Oberflächennachvernetzungsmittel und Sulfinsäure-Derivat beschichtete Superabsorber durchläuft dann die weiteren zur Oberflächennachvernetzung erforderlichen Verfahrensschritte, beispielsweise eine thermisch induzierte Reaktion des Oberflächennachvernetzungsmittels mit dem Superabsorber. Dieses Verfahren ist vergleichsweise einfach und wirtschaftlich.

Falls höchste Stabilität gegen Verfärbung wesentlich ist, wird das Sulfinsäure-Derivat vorzugsweise nach der Oberflächennachvernetzung in einem eigenen Verfahrensschritt aufgebracht. Falls das Sulfinsäure-Derivat als Lösung oder Suspension aufgebracht wird, erfolgt die Aufbringung dabei auf den bereits oberflächennachvernetzten Superabsorber in gleicher Weise wie die Aufbringung des Oberflächennachvernetzungsmittels auf das Grundpolymer. Meist,

aber nicht notwendigerweise wird anschließend ebenso wie bei der Oberflächennachvernetzung erwärmt, um den Superabsorber wieder zu trocknen. Die bei dieser Trocknung eingestellte Temperatur liegt dann aber im Allgemeinen bei höchstens 110 °C, vorzugsweise höchstens 100 °C und in besonders bevorzugter Weise höchstens 90 °C, um unerwünschte Reaktionen des Sulfinsäu-re-Derivats zu vermeiden. Die Temperatur wird so eingestellt, dass in Anbetracht der Verweilzeit im Trocknungsaggregat der gewünschte Wassergehalt des Superabsorbers erreicht wird. Es ist durchaus auch möglich und bequem, das Sulfinsäure-Derivat einzeln oder gemeinsam mit anderen üblichen Hilfsmitteln, beispielsweise Staubbindemitteln, Mitteln gegen Verbackung oder Wasser zur Rückbefeuchtung des Superabsorbers, wie unten für diese Hilfsmittel beschrieben zuzugeben, beispielsweise in einem der Oberflächennachvernetzung nachgeschalteten Kühler. Die Temperatur der Polymerpartikel beträgt in diesem Fall zwischen 0 °C und 190 °C, bevorzugt weniger als 160 °C, mehr bevorzugt weniger als 130 °C, noch mehr bevorzugt weniger als 100 °C, und am meisten bevorzugt weniger als 70 °C. Die Polymerpartikel werden gegebenenfalls nach Beschichtung zügig auf Temperaturen unterhalb der Zersetzungstemperatur des Sulfinsäure-Derivats abgekühlt.

Als an sich bekannter Superabsorber, der durch Einmischung des Sulfinsäure-Derivats und von Phosphinat und/oder Phosphonat oder durch Oberflächennachvernetzung, wobei die Nachvernetzungsreaktion bei einem Partialdruck oxidierender Gase von weniger als 140 mbar durchgeführt wird, zum erfindungsgemäßen Superabsorber wird, kann jeder Superabsorber verwendet werden. Im Allgemeinen sind derartige Superabsorber vernetzte hydrophile Polymere, insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oderStärkeether, vernetzte Carboxy.methylcellulose, teilweise vernetztes Polyalkylenoxid oder in wässrigen Flüssigkeiten quellbare Naturprodukte, wie beispielsweise Guarderivate. Vorzugsweise wird ein Superabsorber auf Basis teilneutralisierter Acrylsäure verwendet. Superabsorber sind vor allem durch ihre Fähigkeit zur Absorption und Retention von Flüssigkeiten gekennzeichnet. Die erfindungsgemäß mit Sulfinsäure-Derivat zu beschichtenden Superabsorber werden so gewählt, dass der fertige Superabsorber eine Zentrifugenretentionskapazität (CRC, Messmethode s. unten) von mindestens 5 g/g, vorzugsweise von mindestens 10 g/g und in besonders bevorzugter Form von mindestens 20 g/g aufweist. Weitere geeignete Mindestwerte der CRC sind beispielsweise 25 g/g, 30 g/g oder 35 g/g. Üblicherweise liegt sie nicht über 40 g/g. Die CRC vieler derzeit marktgängiger und erfindungsgemäß verwendbarer Superabsorber liegt im Bereich von 28 bis 33 g/g.

Die erfindungsgemäß mit Sulfinsäure-Derivat zu beschichtenden Superabsorber werden weiterhin so gewählt, dass der fertige Superabsorber typischerweise eine Absorption unter Druck (AUL0.7psi, Messmethode s. unten) von mindestens 18 g/g, vorzugsweise mindestens 20 g/g, bevorzugt mindestens 22 g/g, besonders bevorzugt mindestens 23 g/g, ganz besonders bevorzugt mindestens 24 g/g und üblicherweise nicht über 30 g/g aufweist.

Die erfindungsgemäß mit Sulfinsäure-Derivat zu beschichtenden Superabsorber werden weiterhin so gewählt, dass der fertige Superabsorber typischerweise eine Flüssigkeitsweiterleitung (SFC, Messmethode s. unten) von mindestens $10x10^{-7}cm^3s/g$, vorzugsweise mindestens $30x10^{-7}cm^3s/g$, bevorzugt mindestens $50x10^{-7}cm^3s/g$, besonders bevorzugt mindestens $80x10^{-7}cm^3s/g$, ganz besonders bevorzugt mindestens $100x10^{-7}cm^3s/g$ und üblicherweise nicht über $1000x10^{-7}cm^3s/g$ aufweist.

CRC, AUL und SFC des Superabsorbers werden im Allgemeinen nicht wesentlich von der Beschichtung des Superabsorbers mit dem Sulfinsäure-Derivat beeinflusst. Diese Parameter werden auf bei der Herstellung von Superabsorbern bekannte und übliche Weise eingestellt. Wenn das Sulfinsäurederivat bei der Oberflächennachvernetzung eines Grundpolymers oder gleichzeitig mit sonstigen Additiven, die diese Eigenschaften von Superabsorbern beeinflussen, zugegeben wird, findet natürlich während der Beschichtung des Superabsorbers mit Sulfinsäurederivat die Einstellung dieser Parameter statt. Diese ist jedoch von diesen anderen Maßnahmen bestimmt und nicht von der Anwesenheit des Sulfinsäurederivats.

Ein erfindungsgemäß mit Sulfinsäurederivat zu beschichtender Superabsorber wird beispielsweise hergestellt durch wässrige Lösungspolymerisation einer Monomermischung, enthaltend

a) mindestens ein ethylenisch ungesättigtes, säuregruppentragendes Monomer, das wahlweise zumindest teilweise als Salz vorliegt,
b) mindestens einen Vernetzer,
c) mindestens einen Initiator,
d) wahlweise ein oder mehrere mit den unter a) genannten Monomeren copolymerisierbare ethylenisch ungesättigte Monomere und
e) wahlweise ein oder mehrere wasserlösliche Polymere.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren oder ihre Salze, wie Acrylsäure, Methacrylsäure, Maleinsäure oder ihre Salze, Maleinsäureanhydrid und Itaconsäure oder ihre Salze. Besonders

bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomerlösung enthält vorzugsweise höchstens 250 Gew.-ppm, bevorzugt höchstens 130 Gew.-ppm, besonders bevorzugt höchstens 70 Gew.-ppm sowie bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a), wobei neutralisiertes Monomer a), d.h. ein Salz des Monomers a) rechnerisch als unneutralisiertes Monomer berücksichtigt wird. Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 530 438 A1 beschrieben, Di- und Triacrylate, wie in EP 547 847 A1, EP 559 476 A1, EP 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/32962 A2 beschrieben.

Bevorzugte Vernetzer b) sind Pentaerythrittriallylether, Tetraallyloxiethan, Methylenbismethacrylamid, 15-bis 20-fach ethoxiliertes Trimethylolpropantriacrylat, 15-20-fach ethoxiliertes Glycerintriacrylat, Polyethylenglykoldiacrylat mit zwischen 4 und 45 - $CH_2CH_2O$-Einheiten in der Molekülkette, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und steigt die Absorption unter einem Druck von 0.3 psi (AUL0.3psi).

Als Initiatoren c) können sämtliche unter den Polymerisationsbedingungen in Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise das oben beschriebene Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt (Brüggolit® FF6M oder Brüggolit® FF7).

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat, Maleinsäure oder ihre Salze und Maleinsäureanhydrid.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. übersättigte Monomerlösungen einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Die Monomermischung kann weitere Komponenten enthalten. Beispiele in derartigen Monomermischungen verwendeter weiterer Komponenten sind etwa Chelatbildner, um Metallionen in Lösung zu halten.

Geeignete Polymerisationsreaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/38402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Fleischwolf, Extruder oder Kneter. Es können aber auch sphärische oder anders geformte Superabsorberpartikel durch Suspensions-, Sprüh- oder Tropfenpolymerisationsverfahren hergestellt werden.

Die Säuregruppen der erhaltenen Polymergele sind üblicherweise teilweise neutralisiert. Die Neutralisation wird vorzugsweise auf der Stufe der Monomeren durchgeführt, mit anderen Worten, es werden Salze der säuregruppentragenden Monomeren oder genaugenommen eine Mischung von säuregruppentragenden Monomeren und Salzen der säuregruppentragenden Monomeren ("teilneutralisierte Säure") als Komponente a) in die Polymerisation eingesetzt. Dies geschieht üblicherweise durch Einmischung des Neutralisationsmittels als wässrige Lösung oder bevorzugt auch als Feststoff in die zur Polymerisation vorgesehene Monomermischung oder bevorzugt in das säuregruppentragende Monomer oder eine Lösung davon. Der Neutralisationsgrad beträgt vorzugsweise von 25 bis 95 mol-%, besonders bevorzugt von 50 bis 80 mol-%, ganz besonders bevorzugt von 65 bis 72 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Es ist jedoch bevorzugt, die Neutralisation auf der Stufe des Monomeren durchzuführen. Mit anderen Worten: in einer ganz besonders bevorzugten Ausführungsform wird als Monomer a) ein Gemisch aus 25 bis 95 mol-%, besonders bevorzugt von 50 bis 80 mol-%, ganz besonders bevorzugt von 65 bis 72 mol-% Salz des säuregruppentragenden Monomeren und dem Rest zu 100 mol-% säuregruppentragendes Monomer eingesetzt. Dieses Gemisch ist beispielsweise ein Gemisch aus Natriumacrylat und Acrylsäure oder ein Gemisch aus Kaliumacrylat und Acrylsäure.

In einer bevorzugten Ausführungsform wird zur Neutralisation ein Neutralisationsmittel verwendet, dessen Gehalt an Eisen im Allgemeinen unter 10 Gew.-ppm, vorzugsweise unter 2 Gew.-ppm und in besonders bevorzugter Weise unter 1 Gew.-ppm liegt. Ebenso ist ein niedriger Gehalt an Chlorid sowie Anionen von Sauerstoffsäuren des Chlors erwünscht. Ein geeignetes Neutralisationsmittel ist beispielsweise die üblicherweise als "membrane grade" gehandelte 50 Gew.-%ige Natronlauge oder Kalilauge, noch reiner und ebenso geeignet, allerdings auch kostspieliger ist die üblicherweise als "amalgame grade" oder "mercury process" gehandelte 50 Gew.-%ige Natronlauge oder Kalilauge.

Das aus der wässrigen Lösungspolymerisation und gegebenenfalls nachträglicher Neutralisation erhaltene Polymergel wird dann vorzugsweise mit einem Bandtrockner getrocknet, bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt (Messmethode für den Restfeuchte- oder Wassergehalt siehe unten). Bei einer zu hohen Restfeuchte weist das getrocknete Polymergel eine zu niedrige Glasübergangstemperatur Tg auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung im Allgemeinen von 25 bis 90 Gew.-%, vorzugsweise von 30 bis 80 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein beheizbarer Mischer mit mechanischem Mischorgan wie beispielsweise ein Schaufeltrockner oder ein ähnlicher Trockner mit anders gestalteten Mischwerkzeugen verwendet werden. Wahlweise kann der Trockner unter Stickstoff oder einem anderen nicht-oxidierenden Inertgas oder zumindest unter verringertem Partialdruck des Sauerstoffs betrieben werden, um oxidative Vergilbungsvorgänge zu verhindern. Im Regelfall führt aber auch eine ausreichende Belüftung und Abführung des Wasserdampfes zu einem akzeptablen Produkt. Vorteilhaft hinsichtlich Farbe und Produktqualität ist in der Regel eine möglichst kurze Trocknungszeit.

Während der Trockung verringert sich auch der Restmonomerengehaltes in den Polymerpartikeln und letzte Reste des Initiators werden zerstört.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen eingesetzt werden können. Übergroße, oft im Inneren noch nicht getrocknete Gelklumpen sind gummielastisch, führen zu Problemen bei der Mahlung und werden vorzugsweise vor der Mahlung abgetrennt, was durch Windsichtung oder ein Sieb ("Schutzsieb" für die Mühle) in einfacher Weise erfolgen kann. Die Maschenweite des Siebs ist angesichts der verwendeten Mühle so zu wählen, dass möglichst keine Störungen durch übergroße, gummielastische Partikel auftreten.

Zu große, nicht ausreichend fein gemahlene Superabsorberpartikel sind bei ihrer überwiegenden Verwendung, in Hygieneprodukten wie Windeln, als grobe Partikel fühlbar, sie senken auch die mittlere Anquellgeschwindigkeit des Superabsorbers Beides ist unerwünscht. Vorteilhafterweise werden daher grobkörnige Polymerpartikel aus dem Produkt abgetrennt. Dies erfolgt durch übliche Klassierverfahren, beispielsweise Windsichtung oder durch Siebung durch ein Sieb mit einer Maschenweite von höchstens 1000 µm, vorzugsweise höchstens 900 µm, besonders bevorzugt höchstens 850 µm und ganz besonders bevorzugt höchstens 800 µm. Beispielsweise werden Siebe mit 700 µm, 650 µm oder 600 µm Maschenweite verwendet. Die abgetrennten grobkörnigen Polymerpartikel ("Überkorn") können zur Kostenoptimierung dem Mahl- und Siebkreislauf wieder zugeführt oder separat weiter verarbeitet werden.

Polymerpartikel mit zu niedriger Partikelgröße senken die Permeabilität (SFC). Vorteilhafterweise werden daher bei dieser Klassierung auch feinkörnige Polymerpartikel abgetrennt. Dies kann, falls gesiebt wird, bequem durch ein Sieb mit einer Maschenweite von höchstens 300 µm, vorzugsweise höchstens 200 µm, in besonders bevorzugter Weise höchstens 150 µm und in ganz besonders bevorzugter Weise höchstens 100 µm verwendet. Die abgetrennten feinkörnigen Polymerpartikel ("Unterkorn" oder "fines") können zur Kostenoptimierung beliebig dem Monomerstrom, dem polymerisierenden Gel, oder dem auspolymerisierten Gel vor der Trocknung des Gels wieder zugeführt werden.

Die mittlere Partikelgröße der als Produktfraktion abgetrennten Polymerpartikel beträgt im Allgemeinen mindestens 200 µm, bevorzugt mindestens 250 µm und in bevorzugter Form mindestens 300 µm sowie im Allgemeinen höchstens 600 µm und in bevorzugter Weise höchstens 500 µm. Der Anteil an Partikeln mit einer Partikelgröße von mindestens 150 µm beträgt im Allgemeinen mindestens 90 Gew.-%, bevorzugterweise mindestens 95 Gew.-% und in besonders bevorzugter Weise mindestens 98 Gew.-%. Der Anteil an Partikeln mit einer Partikelgröße von höchstens 850 µm, beträgt im Allgemeinen mindestens 90 Gew.-%, bevorzugterweise mindestens 95 Gew.-% und in besonders bevorzugter Weise mindestens 98 Gew.-%.

Das so hergestellte Polymer hat superabsorbierende Eigenschaften und fällt unter den Begriff "Superabsorber". Seine CRC ist typischerweise vergleichsweise hoch, seine AUL oder SFC dagegen vergleichsweise niedrig. Ein derartiger, nicht oberflächennachvernetzter Superabsorber wird zur Unterscheidung von einem daraus hergestellten oberflächennachvernetzten Superabsorber oft "Grundpolymer" oder "Basispolymer" genannt. Ein Grundpolymer kann durchaus im Rahmen der vorliegenden Erfindung durch Zusatz von Sulfinsäure-Derivat und von Phosphinat und/oder Phosphonat auch ohne Oberflächennachvernetzung zu einem erfindungsgemäßen Superabsorber verarbeitet werden.

Die Superabsorberpartikel können zur weiteren Verbesserung der Eigenschaften, insbesondere Erhöhung der AUL und SFC-Werte (wobei der CRC-Wert sinkt) an ihrer Oberfläche nachvernetzt werden und der erfindungsgemäße Superabsorber, der oberflächennachvernetzt ist, wobei die Nachvernetzungsreaktion bei einem Partialdruck oxidierender Gase von weniger als 140 mbar durchgeführt wird, wird oberflächennachvernetzt. Geeignete Nachvernetzer sind Verbindungen, die Gruppen enthalten, die mit mindestens zwei funktionellen Gruppen der Superabsorberpartikel Bindungen bilden können. Bei den auf dem Markt vorherrschenden Superabsorbern auf Acrylsäure/Natriumacrylat-Basis sind geeignete Oberflächennachvernetzer Verbindungen, die Gruppen enthalten, die mit mindestens zwei Carboxylatgruppen Bindungen bilden können. Bevorzugte Nachvernetzer sind Amidacetale oder Carbamate der allgemeinen Formel (II)

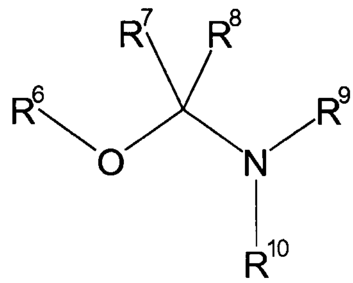

(II)

worin

| | |
|---|---|
| $R^6$ | $C_1$-$C_{12}$-Alkyl, $C_2$-$C_{12}$-Hydroxyalkyl, $C_2$-$C_{12}$-Alkenyl oder $C_6$-$C_{12}$-Aryl, |
| $R^7$ | X oder $OR^{11}$, |
| $R^8$ | Wasserstoff, $C_1$-$C_{12}$-Alkyl, $C_2$-$C_{12}$-Hydroxyalkyl, $C_2$-$C_{12}$-Alkenyl oder $C_6$-$C_{12}$-Aryl, oder X, |
| $R^9$ | $C_1$-$C_{12}$-Alkyl, $C_2$-$C_{12}$-Hydroxyalkyl, $C_2$-$C_{12}$-Alkenyl oder $C_6$-$C_{12}$-Aryl, |
| $R^{10}$ | Wasserstoff, $C_1$-$C_{12}$-Alkyl, $C_2$-$C_{12}$-Hydroxyalkyl, $C_2$-$C_{12}$-Alkenyl, $C_1$-$C_{12}$-Acyl oder $C_6$-$C_{12}$-Aryl, |
| $R^{11}$ | $C_1$-$C_{12}$-Alkyl, $C_2$-$C_{12}$-Hydroxyalkyl, $C_2$-$C_{12}$-Alkenyl oder $C_6$-$C_{12}$-Aryl und |
| X | ein für die Reste $R^7$ und $R^8$ gemeinsamer Carbonylsauerstoff |

bedeuten, wobei $R^6$ und $R^9$ und/oder $R^{10}$ und $R^{11}$ ein verbrücktes $C_2$-$C_6$-Alkandiyl sein können, und wobei die obengenannte Reste $R^6$ bis $R^{11}$ noch insgesamt über ein bis zwei freie Valenzen verfügen können und mit diesen freien Valenzen mit mindestens einem geeigneten Grundkörper verbunden sein können,
oder mehrwertige Alkohole, wobei der mehrwertige Alkohol vorzugsweise ein Molekulargewicht von weniger als 100 g/mol, bevorzugt von weniger als 90 g/mol, besonders bevorzugt von weniger als 80 g/mol, ganz besonders bevorzugt von weniger als 70 g/mol, pro Hydroxygruppe sowie keine vicinalen, geminalen, sekundären oder tertiären Hydroxygruppen aufweist, und mehrwertige Alkohole entweder Diole der allgemeinen Formel (IIIa)

$$HO-R^{12}-OH \qquad \text{(IIIa)}$$

worin $R^{12}$ entweder einen unverzweigten Dialkylrest der Formel $-(CH_2)_n-$, wobei n eine ganze Zahl von 3 bis 20, bevorzugt 3 bis 12 ist, bedeutet und beide Hydroxygruppen endständig sind, oder $R^{12}$ einen unverzweigten, verzweigten oder cyclischen Dialkylrest bedeutet, oder Polyole der allgemeinen Formel (IIIb)

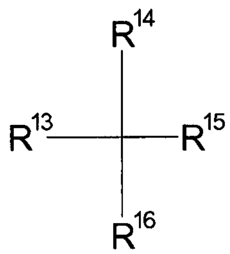

(IIIb)

worin die Reste $R^{13}$, $R^{14}$, $R^{15}$, $R^{16}$ unabhängig voneinander Wasserstoff, Hydroxyl, Hydroxymethyl, Hydroxyethyloxymethyl, 1-Hydroxyprop-2-yloxymethyl, 2-Hydroxypropyloxymethyl, Methyl, Ethyl, n-Propyl, Isopropyl, n-Butyl, n-Pentyl, n-Hexyl, 1,2-Dihydroxyethyl, 2-Hydroxyethyl, 3-Hydroxypropyl oder 4-Hydroxybutyl bedeuten und insgesamt 2, 3, oder 4, bevorzugt 2 oder 3, Hydroxygruppen vorhanden sind, und nicht mehr als einer der Reste $R^{13}$, $R^{14}$, $R^{15}$, oder $R^{16}$ gleich Hydroxyl bedeutet, sind,

oder cyclische Carbonate der allgemeinen Formel (IV)

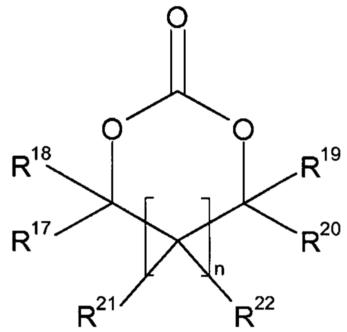

(IV)

worin $R^{17}$, $R^{18}$, $R^{19}$, $R^{20}$, $R^{21}$ und $R^{22}$ unabhängig voneinander Wasserstoff, Methyl, Ethyl, n-Propyl, Isopropyl, n-Butyl, sek.-Butyl oder Isobutyl, und n entweder 0 oder 1 ist,
oder Bisoxazoline der allgemeinen Formel (V)

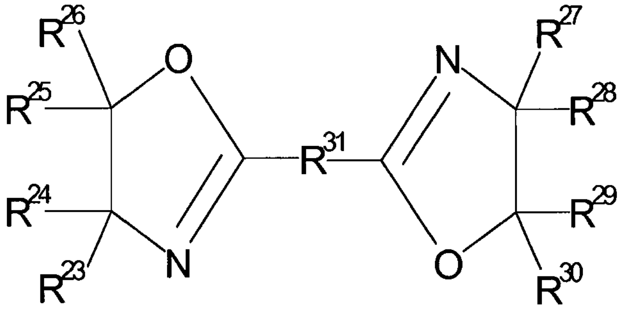

(V)

worin $R^{23}$, $R^{24}$, $R^{25}$, $R^{26}$, $R^{27}$, $R^{28}$, $R^{29}$ und $R^{30}$ unabhängig voneinander Wasserstoff, Methyl, Ethyl, n-Propyl, Isopropyl, n-Butyl, sek.-Butyl oder Isobutyl, und $R^{31}$ eine Einfachbindung, einen linearen, verzweigten oder cyclischen $C_2$-$C_{12}$-Dialkylrest, oder einen Polyalkoxydiylrest darstellt, welcher aus ein bis zehn Ethylenoxid- und/oder Propylenoxideinheiten aufgebaut ist, wie sie beispielsweise Polyglykoldicarbonsäuren aufweisen.

Weitere geeignete Nachvernetzer sind (VI) β-Hydroxialkylamide (beispielsweise das von EMS-Chemie, Reichenauerstrasse, 7013 Domat/Ems, Schweiz, vertriebene Primid® XL-512), (VII) Polyepoxide, (VIII) Polyaziridene und (IX) Oxetanderivate.

Bevorzugte Nachvernetzer der allgemeinen Formel (II) sind 2-Oxazolidone, wie 2-Oxazolidon und N-(2-Hydroxyethyl)-2-oxazolidon, N-Methyl-2-Oxazolidon, N-Acyl-2-oxazolidone, wie N-Acetyl-2-oxazolidon, 2-Oxotetrahydro-1,3-oxazin, bicyclische Amidacetale, wie 5-Methyl-1-aza-4,6-dioxa-bicyclo[3.3.0]octan, 1-Aza-4,6-dioxa-bicyclo[3.3.0]octan und 5-Isopropyl-1-aza-4,6-dioxa-bicyclo [3.3.0] octan, Bis-2-oxazolidone und Poly-2-oxazolidone.

Besonders bevorzugte Nachvernetzer der allgemeinen Formel (II) sind 2-Oxazolidon, N-Methyl-2-oxazolidon, N-(2-Hydroxyethyl)-2-oxazolidon und N-Hydroxypropyl-2-oxazolidon.

Bevorzugte Nachvernetzer der allgemeinen Formel (IIIa) sind 1,3-Propandiol, 1,5-Pentandiol, 1,6-Hexandiol und 1,7-Heptandiol. Weitere Beispiele für Nachvernetzer der Formel (IIIa) sind 1,3-Butandiol, 1,8-Octandiol, 1,9-Nonandiol und 1,10-Decandiol.

Die Diole sind vorzugsweise wasserlöslich, wobei sich die Diole der allgemeinen Formel (IIIa) bei 23°C zu mindestens 30 Gew.-%, bevorzugt zu mindestens 40 Gew.-%, besonders bevorzugt zu mindestens 50 Gew.-%, ganz besonders bevorzugt mindestens zu 60 Gew.-%, in Wasser lösen, wie beispielsweise 1,3-Propandiol und 1,7-Heptandiol. Noch mehr bevorzugt sind solche Nachvernetzer die bei 25°C flüssig sind.

Bevorzugte Nachvernetzer der allgemeinen Formel (IIIb) sind Butan-1,2,3-triol, Butan-1,2,4-triol, Glyzerin, Trimethylolpropan, Trimethylolethan, Pentaerythrit, pro Molekül 1-bis 3-fach ethoxyliertes Glyzerin, Trimethylolethan oder Trimethylolpropan und pro Molekül 1- bis 3-fach propoxyliertes Glyzerin, Trimethylolethan oder Trimethylolpropan. Weiterhin bevorzugt sind 2-fach ethoxyliertes oder propoxyliertes Neopentylglykol. Besonders bevorzugt sind 2-fach und 3-fach ethoxyliertes Glyzerin, Neopentylglykol, 2-Methyl-1,3-propandiol und Trimethylolpropan.

Bevorzugte mehrwertige Alkohole (IIIa) und (IIIb) weisen bei 23 °C eine Viskosität von weniger als 3000 mPas, vorzugsweise weniger als 1500 mPas, bevorzugt weniger als 1000 mPas, besonders bevorzugt weniger als 500 mPas, ganz besonders bevorzugt weniger als 300 mPas, auf.

Besonders bevorzugte Nachvernetzer der allgemeinen Formel (IV) sind Ethylencarbonat und Propylencarbonat.

Ein besonders bevorzugter Nachvernetzer der allgemeinen Formel (V) ist 2,2'-Bis(2-oxazolin).

Die bevorzugten Nachvernetzer minimieren Neben- und Folgereaktionen, die zu flüchtigen und damit übelriechenden Verbindungen führen. Die mit den bevorzugten Nachvernetzern hergestellten wasserabsorbierenden Polymere sind daher auch im angefeuchteten Zustand geruchsneutral. Außerdem sind die bevorzugten Nachvernetzer in besonderem Maße toxisch unbedenklich.

Es kann ein einzelner Nachvernetzer aus der obigen Auswahl verwendet werden oder beliebige Gemische verschiedener Nachvernetzer.

Der Nachvernetzer wird im Allgemeinen in einer Menge von mindestens 0,001 Gew.-%, vorzugsweise von mindestens 0,02 Gew.-%, in besonders bevorzugter Form von mindestens 0,05 Gew.% sowie im Allgemeinen höchstens 2 Gew.-%, vorzugsweise höchstens 1 Gew.-%, in besonders bevorzugter Form höchstens 0,3 Gew.-%, beispielsweise höchstens 0,15 Gew.-% oder höchstens 0,095 Gew.-% eingesetzt, jeweils auf die Masse des Grundpolymeren bezogen.

Die Nachvernetzung wird üblicherweise so durchgeführt, dass eine Lösung des Nachvernetzers auf die getrockneten Grundpolymerpartikel aufgesprüht wird. Im Anschluss an das Aufsprühen werden die mit Nachvernetzer beschichteten Polymerpartikel thermisch getrocknet, wobei die Nachvernetzungsreaktion sowohl vor als auch während der Trocknung stattfinden kann. Falls Oberflächennachvernetzer mit polymerisierbaren Gruppen verwendet werden, kann die Oberflächennachvernetzung auch durch radikalisch induzierte Polymerisation solcher Gruppen mittels gängiger Radikalbildner oder auch mittels energiereicher Strahlung wie beispielsweise UV-Licht erfolgen. Dies kann parallel oder anstatt der Verwendung von Nachvernetzern erfolgen, die kovalente oder ionische Bindungen zu funktionellen Gruppen an der Oberfläche der Grundpolymerpartikel ausbilden.

Das Aufsprühen der Nachvernetzerlösung wird vorzugsweise in Mischern mit bewegten Mischwerkzeugen, wie Schneckenmischern, Scheiben-, Paddel- oder Schaufelmischern oder Mischern mit anderen Mischwerkzeugen durchgeführt. Besonders bevorzugt sind jedoch Vertikalmischer. Es ist aber auch möglich die Nachvernetzerlösung in einem Wirbelbett aufzusprühen. Geeignete Mischer sind beispielsweise als Pflugschar®-Mischer von Gebr. Lödige Maschinenbau GmbH, Elsener-Straße 7 - 9, 33102 Paderborn, Deutschland, oder als Schugi® Flexomix®-Mischer, Vrieco-Nauta®-Mischer oder Turbulizer®-Mischer von Hosokawa Micron BV, Gildenstraat 26, 7000 AB Doetinchem, Niederlande, erhältlich.

Die einsetzbaren Sprühdüsen unterliegen keiner Beschränkung. Geeignete Düsen und Zerstäubungssysteme sind beispielsweise in den folgenden Literaturstellen beschrieben: Zerstäuben von Flüssigkeiten, Expert-Verlag, Bd. 660, Reihe Kontakt & Studium, Thomas Richter (2004) sowie in Zerstäubungstechnik, Springer-Verlag, VDI-Reihe, Günter Wozniak (2002). Einsetzbar sind mono- und polydisperse Sprühsysteme. Unter den polydispersen Systemen sind Einstoff-Druckdüsen (strahl- oder lamellenbildend), Rotationszerstäuber, Zweistoffzerstäuber, Ultraschallzerstäuber und Pralldüsen geeignet. Bei den Zweistoffzerstäubern kann die Mischung der Flüssigkeits- mit der Gasphase sowohl innenliegend als auch außenliegend erfolgen. Das Sprühbild der Düsen ist unkritisch und kann jede beliebige Form annehmen, beispielsweise Rundstrahl-, Flachstrahl-, Weitwinkel-Rundstrahl- oder Kreisring-Spritzbild. Vorteilhaft ist die Verwendung eines nicht-oxidierenden Gases, falls Zweistoffzerstäuber eingesetzt werden, besonders bevorzugt sind Stickstoff, Argon oder Kohlendioxid. Derartigen Düsen kann die zu versprühende Flüssigkeit unter Druck zugeführt werden. Die Zerteilung der zu versprühenden Flüssigkeit kann dabei dadurch erfolgen, dass sie nach Erreichen einer bestimmten Mindestgeschwindigkeit in der Düsenbohrung entspannt wird. Ferner können für den erfindungsgemäßen Zweck auch Einstoffdüsen, wie beispielsweise Schlitzdüsen oder Drallkammern (Vollkegeldüsen) verwendet werden (beispielsweise von Düsen-Schlick GmbH, DE, oder von Spraying Systems Deutschland GmbH, DE). Derartige Düsen sind auch in der EP 0 534 228 A1 und der EP 1 191 051 A2 beschrieben.

Die Nachvernetzer werden typischerweise als wässrige Lösung eingesetzt. Wird ausschließlich Wasser als Lösungsmittel verwendet, so wird der Nachvernetzerlösung oder bereits dem Grundpolymer vorteilhafterweise ein Tensid oder Deagglomerisationshilfsmittel zugesetzt. Dadurch wird das Benetzungsverhalten verbessert und die Verklumpungsneigung vermindert.

Alle anionischen, kationischen, nichtionischen und amphoteren Tenside sind als Deagglomerationshilfsmittel geeignet, bevorzugt sind jedoch aus Hautverträglichkeitsgründen nicht-ionische und amphotere Tenside. Das Tensid kann auch Stickstoff enthalten. Beispielsweise werden Sorbitanmonoester, wie Sorbitanmonococoat und Sorbitanmonolaurat, oder ethoxylierte Varianten davon, wie beispielsweise Polysorbat 20®, zugesetzt. Weitere geeignete Deagglomerationshilfsmittel stellen die ethoxylierten und alkoxylierten Derivate des 2-Propylheptanols dar, die unter den Marken Lutensol XL® und Lutensol XP® vertrieben werden (BASF SE, Carl-Bosch-Straße 38, 67056 Ludwigshafen, Deutschland).

Das Deagglomerationshilfsmittel kann getrennt dosiert oder der Nachvernetzerlösung zugesetzt werden. Vorzugsweise wird das Deagglomerationshilfsmittel der Nachvernetzerlösung einfach zugesetzt.

Die Einsatzmenge des Deagglomerationshilfsmittels bezogen auf Grundpolymer beträgt beispielsweise 0 bis 0,1 Gew.-%, vorzugsweise 0 bis 0,01 Gew.-%, besonders bevorzugt 0 bis 0,002 Gew.-%. Vorzugsweise wird das Deagglomerationshilfsmittel so dosiert, dass die Oberflächenspannung eines wässrigen Extrakts des gequollenen Grundpolymers und/oder des gequollenen nachvernetzten wasserabsorbierenden Polymeren bei 23 °C mindestens

0,060 N/m, vorzugsweise mindestens 0,062 N/m, besonders bevorzugt mindestens 0,065 N/m, und vorteilhaft höchstens 0,072 N/m beträgt.

Die wässrige Nachvernetzerlösung kann neben dem mindestens einen Nachvernetzer auch noch ein Cosolvens enthalten. Über den Gehalt an nichtwässrigem Lösungsmittel bzw. Gesamtlösungsmittelmenge kann die Eindringtiefe des Nachvernetzers in die Polymerpartikel eingestellt werden. Technisch gut geeignete Cosolventien sind C1-C6-Alkohole, wie Methanol, Ethanol, n-Propanol, Isopropanol, n-Butanol, sec-Butanol, tert.-Butanol oder 2-Methyl-1-propanol, C2-C5-Diole, wie Ethylenglykol, 1,2-Propylenglykol oder 1,4-Butandiol, Ketone, wie Aceton, oder Carbonsäureester, wie Essigsäureethylester. Nachteilig an einigen dieser Cosolventien ist, dass sie typische Eigengerüche aufweisen.

Das Cosolvens selbst ist unter den Reaktionsbedingungen idealerweise kein Nachvernetzer. Es kann jedoch im Grenzfall und abhängig von Verweilzeit und Temperatur dazu kommen, dass das Cosolvens teilweise zur Vernetzung beiträgt. Dies ist insbesondere dann der Fall, wenn der Nachvernetzer relativ träge ist und daher auch selbst sein Cosolvens bilden kann, wie beispielsweise bei Einsatz cyclischer Carbonate der allgemeinen Formel (IV), Diole der allgemeinen Formel (IIIa) oder Polyole der allgemeinen Formel (IIIb). Solche Nachvernetzer können im Gemisch mit reaktiveren Nachvernetzern auch in der Funktion als Cosolvens eingesetzt werden, da die eigentliche Nachvernetzungsreaktion dann bei niedrigeren Temperaturen und/oder kürzeren Verweilzeiten als in Abwesenheit des reaktiveren Vernetzers durchgeführt werden kann. Da das Cosolvens in relativ großen Mengen verwendet wird und auch teilweise im Produkt verbleibt, darf es nicht toxisch sein.

In den erfindungsgemäßen Verfahren eignen sich die Diole der allgemeinen Formel (IIIa), die Polyole der allgemeinen Formel (IIIb), sowie die cyclischen Carbonate der allgemeinen Formel (IV) auch als Cosolventien. Diese Funktion erfüllen sie in Gegenwart eines reaktiven Nachvernetzers der allgemeinen Formel (II) und/oder (V) und/oder einer Di- der Triglycidylverbindung. Bevorzugte Cosolventien in den erfindungsgemäßen Verfahren sind jedoch insbesondere die Diole der allgemeinen Formel (IIIa), insbesondere, wenn die Hydroxygruppen sterisch durch Nachbargruppen an einer Reaktion behindert werden. Solche Diole eignen sich zwar prinzipiell auch als Nachvernetzer, erfordern dazu jedoch deutlich höhere Reaktionstemperaturen oder gegebenenfalls höhere Einsatzmengen als sterisch ungehinderte Diole.

Besonders bevorzugte Kombinationen aus wenig reaktivem Nachvernetzer als Cosolvens und reaktivem Nachvernetzer sind Kombinationen von bevorzugten mehrwertigen Alkoholen, Diolen der allgemeinen Formel (IIIa) und Polyolen der allgemeinen Formel (IIIb), mit Amidacetalen oder Carbamaten der allgemeinen Formel (II).

Geeignete Kombinationen sind beispielsweise 2-Oxazolidon/1,2-Propandiol und N-(2-Hydroxyethyl)-2-oxazolidon/1,2-Propandiol sowie Ethylenglykoldiglycidylether/1,2-Propandiol.

Ganz besonders bevorzugte Kombinationen sind 2-Oxazolidon/1,3-Propandiol und N-(2-Hydroxyethyl)-2-oxazolidon/1,3-Propandiol.

Weiterhin bevorzugte Kombinationen sind solche mit Ethylenglykoldiglycidylether oder Glyzerindi- oder -triglycidylether mit folgenden Lösemitteln, Cosolventien oder Covernetzern: Isopropanol, 1,3-Propandiol, 1,2-Propylenglykol oder Gemischen davon.

Weiterhin bevorzugte Kombinationen sind solche mit 2-Oxazolidon oder (2-Hydroxyethyl)-2-oxazolidon in folgenden Lösemitteln, Cosolventien oder Covernetzern: Isopropanol, 1,3-Propandiol, 1,2-Propylenglykol, Ethylencarbonat, Propylencarbonat oder Gemischen davon.

Häufig beträgt die Konzentration des Cosolvens in der wässrigen Nachvernetzerlösung, von 15 bis 50 Gew.-%, vorzugsweise von 15 bis 40 Gew.-%, besonders bevorzugt von 20 bis 35 Gew.-%, bezogen auf die Nachvernetzerlösung. Bei Cosolventien, die mit Wasser nur begrenzt mischbar sind, wird man vorteilhaft die wässrige Nachvernetzerlösung so einstellen, dass nur eine Phase vorliegt, gegebenenfalls durch Erniedrigung der Konzentration des Cosolvens.

In einer bevorzugten Ausführungsform wird kein Cosolvens eingesetzt. Der Nachvernetzer wird dann nur als Lösung in Wasser angewandt, gegebenenfalls unter Zusatz eines Deagglomerationshilfsmittels.

Die Konzentration des mindestens einen Nachvernetzers in der wässrigen Nachvernetzerlösung, beträgt typischerweise 1 bis 20 Gew.-%, vorzugsweise 1,5 bis 10 Gew.-%, besonders bevorzugt 2 bis 5 Gew.-%, bezogen auf die Nachvernetzerlösung.

Die Gesamtmenge der Nachvernetzerlösung bezogen auf Grundpolymer beträgt üblicherweise von 0,3 bis 15 Gew.-%, vorzugsweise von 2 bis 6 Gew.-%.

Die eigentliche Oberflächennachvernetzung durch Reaktion des Oberflächennachvernetzers mit funktionellen Gruppen an der Oberfläche der Grundpolymerpartikel wird meist durch Erwärmung des mit Oberflächennachvernetzerlösung benetzten Grundpolymers durchgeführt, üblicherweise "Trocknung" genannt (aber nicht mit der oben beschriebenen Trocknung des Polymergels aus der Polymerisation zu verwechseln, bei der typischerweise sehr viel mehr Flüssigkeit zu entfernen ist). Die Trocknung kann im Mischer selbst erfolgen, durch Beheizung des Mantels, durch Wärmeaustauschflächen oder Einblasen warmer Gase. Gleichzeitiges Versetzen des Superabsorbers mit Oberflächennachvernetzer und Trocknen kann beispielsweise in einem Wirbelschichttrockner erfolgen. Die Trocknung wird aber meist in einem nachgeschalteten Trockner, wie beispielsweise einem Hordentrockner, einem Drehrohrofen, ein Paddel- oder Scheibentrockner oder einer beheizbaren Schnecke durchgeführt. Geeignete Trockner sind beispielsweise als Solidair®

oder Torusdisc®-Trockner von Bepex International LLC, 333 N.E. Taft Street, Minneapolis, MN 55413, U.S.A., oder als Paddel- oder Schaufeltrockner oder auch als Fließbetttrockner von Nara Machinery Co., Ltd., Zweigniederlassung Europa, Europaallee 46, 50226 Frechen, Deutschland erhältlich.

Es ist möglich die Polymerpartikel zur Trocknung und Durchführung der Oberflächennachvernetzung über Kontaktflächen in einem nachgeschalteten Trockner zu beheizen, oder über zugeführtes warmes Inertgas, oder über eine Mischung eines oder mehrerer Inertgase mit Wasserdampf, oder nur mit Wasserdampf allein. Bei Zufuhr der Wärme über Kontaktflächen ist es möglich die Reaktion bei leichtem oder vollständigem Unterdruck unter Inertgas durchzuführen. Bei Verwendung von Wasserdampf zum direkten Beheizen der Polymerpartikel ist es erfindungsgemäß wünschenswert den Trockner bei Normaldruck oder Überdruck zu betreiben. In diesem Fall kann es sinnvoll sein den Nachvernetzungsschritt in einen Aufheizschritt mit Wasserdampf und einen Reaktionsschritt unter Inertgas aber ohne Wasserdampf aufzuspalten. Dies kann in einem oder mehreren Apparaten realisiert werden. Erfindungsgemäß können die Polymerpartikel schon im Nachvernetzungsmischer mit Wasserdampf aufgeheizt werden. Das eingesetzte Grundpolymer kann aus vorhergehenden Prozeßschritten noch eine Temperatur von 10 bis 120 °C aufweisen, die Nachvernetzerlösung kann eine Temperatur von 0 bis 70 °C aufweisen. Insbesondere kann die Nachvernetzerlösung zur Verminderung der Viskosität erwärmt werden.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten. Typischerweise wird die Trocknung so geführt, dass der Superabsorber einen Restfeuchtegehalt von im Allgemeinen mindestens 0,1 Gew.-%, vorzugsweise mindestens 0,2 Gew.-% und in besonders bevorzugter Form mindestens 0,5 Gew.-% sowie im Allgemeinen höchstens 15 Gew.-%, vorzugsweise höchstens 10 Gew.-% und in besonders bevorzugter Form höchstens 8 Gew.-% aufweist.

Die Nachvernetzung kann unter normalen atmosphärischen Bedingungen stattfinden. Normale atmosphärische Bedingungen bedeutet, dass keine technischen Vorkehrungen getroffen werden, um den Partialdruck oxidierender Gase wie den des atmosphärischen Sauerstoffs im Apparat, in dem die Nachvernetzungsreaktion überwiegend stattfindet (dem "Nachvernetzungsreaktor", typischerweise der Trockner) zu verringern. Es ist jedoch bevorzugt, die Nachvernetzungsreaktion unter verringertem Partialdruck oxidierender Gase durchzuführen. Oxidierende Gase sind Stoffe, die bei 23 °C einen Dampfdruck von mindestens 1013 mbar aufweisen und in Verbrennungsvorgängen als Oxidationsmittel wirken, beispielsweise Sauerstoff, Stickoxid und Stickstoffdioxid, insbesondere Sauerstoff. Vorzugsweise beträgt der Partialdruck oxidierender Gase dabei weniger als 140 mbar, bevorzugt weniger als 100 mbar, besonders bevorzugt weniger als 50 mbar, ganz besonders bevorzugt weniger als 10 mbar und der erfindungsgemäße Superabsorber, der oberflächennachvernetzt ist, wobei die Nachvernetzungsreaktion bei einem Partialdruck oxidierender Gase von weniger als 140 mbar durchgeführt wird, wird bei diesem Partialdruck oberflächennachvernetzt. Wird die thermische Nachvernetzung bei Umgebungsdruck, d. h. bei einem Gesamtdruck um 1013 mbar, durchgeführt, so wird der Gesamtpartialdruck der oxidierenden Gase über deren Volumenanteil festgelegt. Der Anteil der oxidierenden Gase beträgt dabei vorzugsweise weniger als 14 Vol.-%, bevorzugt weniger als 10 Vol.-%, besonders bevorzugt weniger als 5 Vol.-%, ganz besonders bevorzugt weniger als 1 Vol.-%.

Die Nachvernetzung kann unter vermindertem Druck durchgeführt werden, d. h. bei einem Gesamtdruck von weniger als 1.013 mbar. Der Gesamtdruck beträgt typischerweise weniger als 670 mbar, vorzugsweise weniger als 480 mbar, besonders bevorzugt weniger als 300 mbar, ganz besonders bevorzugt weniger als 200 mbar. Werden Trocknung und Nachvernetzung unter Luft mit einem Sauerstoffgehalt von 20,8 Vol.-% durchgeführt, so betragen die zu den obengenannten Gesamtdrücken korrespondierenden Sauerstoffpartialdrücke 139 mbar (670 mbar), 100 mbar (480 mbar), 62 mbar (300 mbar) und 42 mbar (200 mbar), wobei die jeweiligen Gesamtdrücke in den Klammern stehen. Eine andere Möglichkeit, den Partialdruck oxidierender Gase zu senken, ist die Einleitung von nicht oxidierenden Gase, insbesondere Inertgasen in den zur Nachvernetzung verwendeten Apparat. Geeignete Inertgase sind bei der Nachvernetzungstemperatur und gegebenem Druck im Nachvernetzungstrockner gasförmig vorliegende Stoffe, die unter diesen Bedingungen nicht oxidierend auf die Bestandteile der trocknenden Polymerpartikel wirken, beispielsweise Stickstoff, Kohlendioxid, Argon, Wasserdampf, wobei Stickstoff bevorzugt ist. Die Inertgasmenge beträgt im Allgemeinen von 0,0001 bis 10 $m^3$, bevorzugt 0,001 bis 5 $m^3$, besonders bevorzugt 0,005 bis 1 $m^3$, und ganz besonders bevorzugt 0,005 bis 0,1 $m^3$, bezogen auf 1 kg Superabsorber.

In den erfindungsgemäßen Verfahren kann das Inertgas, wenn es nicht Wasserdampf enthält, über Düsen in den Nachvernetzungstrockner eingeblasen werden, besonders bevorzugt wird das Inertgas aber bereits im oder kurz vor dem Mischer, indem der Superabsorber mit Oberflächennachvernetzer versetzt wird, über Düsen dem Polymerpartikelstrom zugegeben.

Selbstverständlich können aus dem Trockner abgeführte Dämpfe von Cosolventien außerhalb des Trockners wieder kondensiert und gegebenenfalls rezykliert werden.

In einer bevorzugten Ausführungsform der vorliegenden Erfindung werden vor, während oder nach der Nachvernetzung zusätzlich zu den Nachvernetzern polyvalente Kationen auf die Partikeloberfläche aufgebracht. Dies ist im Prinzip eine weitere Oberflächennachvernetzung durch ionische, nicht kovalente Bindungen, wird aber gelegentlich auch als "Komplexierung" mit den betreffenden Metallionen oder einfach als "Beschichtung" mit den betreffenden Substanzen (dem "Komplexierungsmittel") bezeichnet.

Dieses Aufbringen von polyvalenten Kationen erfolgt durch Aufsprühen von Lösungen zwei- oder mehrwertiger Kationen, meist zwei-, drei- oder vierwertiger Metallkationen, aber auch polyvalenter Kationen wie formal ganz oder teilweise aus Vinylaminmonomeren aufgebauter Polymere wie teilweise oder vollständig hydrolysiertes Polyvinylamid (sogenanntes "Polyvinylamin"), dessen Amingruppen stets - auch bei sehr hohen pH-Werten - teilweise zu Ammoniumgruppen protoniert vorliegen. Beispiele verwendbarer zweiwertiger Metallkationen sind insbesondere die zweiwertigen Kationen von Metallen der Gruppen 2 (insbesondere Mg, Ca, Sr, Ba), 7 (insbesondere Mn), 8 (insbesondere Fe), 9 (insbesondere Co), 10 (insbesondere Ni), 11 (insbesondere Cu) und 12 (insbesondere Zn) des Periodensystems der Elemente. Beispiele verwendbarer dreiwertiger Metallkationen sind insbesondere die dreiwertigen Kationen von Metallen der Gruppen 3 einschließlich der Lanthaniden (insbesondere Sc, Y, La, Ce), 8 (insbesondere Fe), 11 (insbesondere Au) und 13 (insbesondere Al) des Periodensystems der Elemente. Beispiele verwendbarer vierwertiger Kationen sind insbesondere die vierwertigen Kationen von Metallen der Lanthaniden (insbesondere Ce) sowie der Gruppe 4 (insbesondere Ti, Zr, Hf) des Periodensystems der Elemente. Die Metallkationen können sowohl allein als auch im Gemisch untereinander eingesetzt werden. Besonders bevorzugt ist die Verwendung dreiwertiger Metallkationen. Ganz besonders bevorzugt ist die Verwendung von Aluminiumkationen.

Von den genannten Metall-Kationen sind alle Metallsalze geeignet, die eine ausreichende Löslichkeit in dem zu verwendenden Lösungsmittel besitzen. Besonders geeignet sind Metallsalze mit schwach komplexierenden Anionen wie zum Beispiel Chlorid, Nitrat und Sulfat, Hydrogensulfat, Carbonat, Hydrogencarbonat, Nitrat, Phosphat, Hydrogenphosphat, oder Dihydrogenphosphat, Bevorzugt sind Salze von Mono- und Dicarbonsäuren, Hydroxisäuren, Ketosäuren sowie Aminosäuren oder basische Salze. Beispielsweise werden Acetate, Propionate, Tartrate, Maleate, Citrate, Laktate, Malate, Succinate. Ebenso bevorzugt ist die Verwendung von Hydroxiden. Besonders bevorzugt ist die Verwendung von 2-Hydroxicarbonsäuresalzen wie Citraten und Laktaten. Beispiele besonders bevorzugter Metallsalze sind Alkali- und Erdalkalimetallaluminate und deren Hydrate, etwa Natriumaluminat und dessen Hydrate, Aluminiumacetat, Aluminiumpropionat, Aluminiumcitrat und Aluminiumlaktat.

Die genannten Kationen und Salze können in Reinform oder als Gemisch verschiedener Kationen oder Salze verwendet werden. Die eingesetzten Salze des zwei und/oder dreiwertigen Metallkations können weitere Nebenbestandteile wie noch unneutralisierte Carbonsäure und/oder Alkalisalze der neutralisierten Carbonsäure enthalten. Bevorzugte Alkalisalze sind die des Natriums, Kaliums und des Ammoniums. Sie werden typischerweise als wässerige Lösung eingesetzt welche durch Auflösen der festen Salze in Wasser gewonnen wird, oder bevorzugt direkt als solche erzeugt wird, wodurch gegebenenfalls Trocknungs- und Reinigungsschritte vermieden werden. Vorteilhaft können auch die Hydrate der genannten Salze eingesetzt werden, die sich oft schneller in Wasser lösen als die wasserfreien Salze.

Die Einsatzmenge an Metallsalz beträgt im Allgemeinen mindestens 0,001 Gew.-%, vorzugsweise mindestens 0,01 Gew.-% und in besonders bevorzugter Form mindestens 0,1 Gew.-%, beispielsweise mindestens 0,4 Gew.-% sowie im Allgemeinen höchstens 5 Gew.-%, vorzugsweise höchstens 2,5 Gew.-% und in besonders bevorzugter Form höchstens 1 Gew.-%, beispielsweise höchstens 0,7 Gew.-% jeweils bezogen auf die Masse des Grundpolymeren.

Das Salz des dreiwertigen Metallkations kann als Lösung oder Suspension eingesetzt werden. Als Lösungsmittel für die Metallsalze können Wasser, Alkohole, DMF, DMSO sowie Mischungen dieser Komponenten eingesetzt werden. Besonders bevorzugt sind Wasser und Wasser/Alkohol-Mischungen wie zum Beispiel Wasser/Methanol, Wasser/1,2-Propandiol und Wasser/1,3-Propandiol.

Die Behandlung des Grundpolymeren mit Lösung eines zwei- oder mehrwertigen Kations erfolgt in gleicher Weise wie die mit Oberflächennachvernetzer, einschließlich des Trocknungsschritts. Oberflächennachvernetzer und polyvalentes Kation können in einer gemeinsamen Lösung oder als getrennte Lösungen aufgesprüht werden. Das Aufsprühen der Metallsalz-Lösung auf die Superabsorberpartikel kann sowohl vor als auch nach der Oberflächennachvernetzung erfolgen. In einem besonders bevorzugten Verfahren erfolgt die Aufsprühung der Metallsalz-Lösung im gleichen Schritt mit dem Aufsprühen der Vernetzer-Lösung, wobei beide Lösungen getrennt nacheinander oder gleichzeitig über zwei Düsen aufgesprüht werden, oder Vernetzer- und Metallsalz-Lösung vereint über eine Düse aufgesprüht werden können.

Insbesondere wenn ein drei- oder höhervalentes Metallkation wie Aluminium zur Komplexierung verwendet wird, wird wahlweise auch ein basisches Salz eines zweiwertigen Metallkations oder ein Gemisch solcher Salze zugegeben. Basische Salze sind Salze, die geeignet sind den pH-Wert einer sauren wässrigen Lösung zu erhöhen, vorzugsweise einer 0,1 N Salzsäure. Basische Salze sind üblicherweise Salze einer starken Base mit einer schwachen Säure.

Das zweiwertige Metallkation des optionalen basischen Salzes ist vorzugsweise ein Metallkation der Gruppe 2 des Periodensystems der Elemente, besonders bevorzugt Calcium oder Strontium, ganz besonders bevorzugt Calcium.

Die basischen Salze der zweiwertigen Metallkationen sind vorzugsweise Salze schwacher anorganischer Säuren, schwacher organischer Säuren und/oder Salze von Aminosäuren, besonders bevorzugt Hydroxide, Hydrogencarbonate, Carbonate, Acetate, Propionate, Citrate, Glukonate, Laktate, Tartrate, Malate, Succinate, Maleate und/oder Fumarate, ganz besonders bevorzugt Hydroxide, Hydrogencarbonate, Carbonate, Propionate und/oder Laktate. Das basische Salz ist vorzugsweise wasserlöslich. Wasserlösliche Salze sind Salze, welche bei 20 °C eine Wasserlöslichkeit von mindestens 0,5 g Salz pro Liter Wasser, vorzugsweise mindestens 1 g Salz pro l Wasser, bevorzugt mindestens 10 g Salz pro l Wasser, besonders bevorzugt mindestens 100 g Salz pro l Wasser, ganz besonders bevorzugt mindestens 200 g Salz pro l Wasser, aufweisen. Erfindungsgemäß verwendbar sind jedoch auch solche Salze, die diese Mindestlöslichkeit bei der Aufsprühtemperatur der Sprühlösung aufweisen. Vorteilhaft können auch die Hydrate der genannten Salze eingesetzt werden, die sich oft schneller in Wasser lösen als die wasserfreien Salze.

Geeignete basische Salze zweiwertiger Metallkationen sind beispielsweise Calciumhydroxid, Strontiumhydroxid, Calciumhydrogencarbonat, Strontiumhydrogencarbonat, Calciumacetat, Strontiumacetat, Calciumpropionat, Strontiumpropionat, Calciumlaktat, Strontiumlaktat, Calciumcarbonat und Strontiumcarbonat.

Wenn die Wasserlöslichkeit nicht ausreicht eine Sprühlösung der gewünschten Konzentration herzustellen, dann können auch Dispersionen des festen Salzes in seiner gesättigten wässerigen Lösung eingesetzt werden. Beispielsweise können Calciumcarbonat, Strontiumcarbonat, Calciumsulfit, Strontiumsulfit, Calciumphosphat und Strontiumphosphat als wässrige Dispersionen eingesetzt werden.

Die Menge an basischem Salz des zweiwertigen Metallkations, bezogen auf die Masse des Grundpolymers, beträgt typischerweise von 0,001 bis 5 Gew.-%, vorzugsweise von 0,01 bis 2,5 Gew.-%, bevorzugt 0,1 bis 1,5 Gew.-%, besonders bevorzugt von 0,1 bis 1 Gew.-%, ganz besonders bevorzugt von 0,4 bis 0,7 Gew.-%.

Das basische Salz des zweiwertigen Metallkations kann als Lösung oder Suspension eingesetzt werden. Beispiele hierfür sind Calciumlaktat-Lösungen oder Calciumhydroxid-Suspensionen. Üblicherweise werden die Salze mit einer Wassermenge von nicht mehr als 15 Gew.-% vorzugsweise von nicht mehr als 8 Gew.-%, besonders bevorzugt von nicht mehr als 5 Gew.-%, ganz besonders bevorzugt von nicht mehr als 2 Gew.-% bezogen auf den Superabsorber aufgesprüht.

Vorzugsweise wird eine wässrige Lösung des basischen Salzes auf den Superabsorber aufgesprüht. Bequemerweise wird das basische Salz gleichzeitig mit dem Oberflächennachvernetzungsmittel, dem Komplexierungsmittel oder als weiterer Bestandteil der Lösungen dieser Mittel zugegeben. Für diese basischen Salze ist die Zugabe im Gemisch mit dem Komplexierungsmittel bevorzugt. Wenn die Lösung des basischen Salzes nicht mit der Lösung des Komplexierungsmittels ohne Ausfällung mischbar ist, so können die Lösungen separat nacheinander oder zeitgleich aus zwei Düsen aufgesprüht werden.

Sofern im Anschluss an die Oberflächennachvernetzung und/oder Behandlung mit Komplexbildner ein Trocknungsschritt durchgeführt wird, ist es vorteilhaft, aber nicht unbedingt notwendig, das Produkt nach der Trocknung zu kühlen. Die Kühlung kann kontinuierlich oder diskontinuierlich erfolgen, bequemerweise wird das Produkt dazu kontinuierlich in einen dem Trockner nachgeschalteten Kühler gefördert. Dazu kann jeder zur Abfuhr von Wärme aus pulverförmigen Feststoffen bekannte Apparat verwendet werden, insbesondere jede oben als Trocknungsapparat erwähnte Vorrichtung, sofern sie nicht mit einem Heizmedium, sondern mit einem Kühlmedium wie etwa mit Kühlwasser beaufschlagt wird, so dass über die Wände und je nach Konstruktion auch über die Rührorgane oder sonstige Wärmeaustauschflächen keine Wärme in den Superabsorber eingetragen, sondern daraus abgeführt wird. Bevorzugt ist die Verwendung von Kühlern, in denen das Produkt bewegt wird, also gekühlten Mischern. beispielsweise Schaufelkühlern, Scheibenkühlern oder Paddelkühlern. Der Superabsorber kann auch in der Wirbelschicht durch Einblasen eines gekühlten Gases wie kalter Luft gekühlt werden. Die Bedingungen der Kühlung werden so eingestellt, dass ein Superabsorber mit der für die Weiterverarbeitung gewünschten Temperatur erhalten wird. Typischerweise wird eine mittlere Verweilzeit im Kühler von im Allgemeinen mindestens 1 Minute, vorzugsweise mindestens 3 Minuten und in besonders bevorzugter Form mindestens 5 Minuten sowie im Allgemeinen höchstens 6 Stunden, vorzugsweise höchstens 2 Stunden und in besonders bevorzugter Weise höchstens 1 Stunde eingestellt und die Kühlleistung so bemessen, dass das erhaltene Produkt eine Temperatur von im Allgemeinen mindestens 0 °C, vorzugsweise mindestens 10 °C und in besonders bevorzugter Form mindestens 20 °C sowie im Allgemeinen höchstens 100 °C, vorzugsweise höchstens 80 °C und in besonders bevorzugter Form höchstens 60 °C aufweist.

Der oberflächennachvernetzte Superabsorber wird wahlweise in üblicher Weise gemahlen und/oder gesiebt. Mahlung ist hier typischerweise nicht erforderlich, meist ist aber zur Einstellung der gewünschten Partikelgrößenverteilung des Produkts das Absieben von gebildeten Agglomeraten oder Feinkorn angebracht. Agglomerate und Feinkorn werden entweder verworfen oder vorzugsweise in bekannter Weise und an geeigneter Stelle in das Verfahren zurückgeführt; Agglomerate nach Zerkleinerung. Die für oberflächennachvernetzte Superabsorber gewünschten Partikelgrößen sind die gleichen wie bei Grundpolymeren.

Wahlweise können auf die Oberfläche der Superabsorberpartikel, ob nicht nachvernetzt oder nachvernetzt, im Herstellverfahren in jedem Prozessschritt bei Bedarf alle bekannten Beschichtungen, wie filmbildende Polymere, thermoplastische Polymere, Dendrimere, polykationische Polymere (wie beispielsweise Polyvinylamin, Polyethylenimin oder Polyallylamin), wasserunlösliche polyvalente Metallsalze, wie beispielsweise Magnesiumcarbonat, Magnesiumoxid, Magnesiumhydroxid, Calciumcarbonat, Calciumsulfat oder Calciumphosphat, alle dem Fachmann bekannten wasserlöslichen mono- oder polyvalenten Metallsalze, wie beispielsweise Aluminiumsulfat, Natrium-, Kalium-, Zirkonium- oder Eisensalze, oder hydrophile anorganische Partikel, wie Tonminerale, pyrogene Kieselsäure, kolloidale Kieselsäuresole wie z.B. Levasil®, Titandioxid, Aluminiumoxid und Magnesiumoxid, zusätzlich aufgebracht werden. Beispiele für nützliche Alkalimetallsalze sind Natrium- und Kaliumsulfat, Natrium- und Kaliumlaktate, - citrate, -sorbate. Dadurch können zusätzliche Effekte, beispielsweise eine verringerte Verbackungsneigung des Endprodukts oder des Zwischenprodukts im jeweiligen Prozessschritt des Herstellverfahrens, verbesserte Verarbeitungseigenschaften oder eine weiter gesteigerte Flüssigkeitsleitfähigkeit (SFC) erreicht werden. Wenn die Additive in Form von Dispersionen eingesetzt und aufgesprüht werden, dann werden sie bevorzugt als wässrige Dispersionen eingesetzt, und es wird bevorzugt noch zusätzlich ein Entstaubungsmittel zur Fixierung des Additivs auf der Oberfläche des wasserabsorbierenden Polymers aufgebracht. Das Entstaubungsmittel wird dann entweder direkt der Dispersion des anorganischen pulvrigen Additivs hinzugefügt, optional kann es auch als separate Lösung vor, während, oder nach dem Auftrag des anorganischen pulvrigen Additivs durch Aufsprühen hinzugefügt werden. Am meisten bevorzugt ist die gleichzeitige Aufsprühung von Nachvernetzungsmittel, Entstaubungsmittel und pulvrigem anorganischen Additiv in der Nachvernetzung. In einer weiteren bevorzugten Verfahrensvariante wird das Entstaubungsmittel aber separat im Kühler zugegeben, beispielsweise durch Aufsprühen von oben, unten oder von der Seite. Besonders geeignete Entstaubungsmittel, die auch zur Fixierung pulvriger anorganischer Additive an der Oberfläche der wasserabsorbierenden Polymerpartikel dienen können, sind Polyethylenglykole mit einem Molekulargewicht von 400 bis 20000 g/mol, Polyglyzerin, 3-bis 100-fach ethoxylierte Polyole, wie Trimethylolpropan, Glyzerin, Sorbitol und Neopentylglykol. Besonders geeignet sind 7- bis 20-fach ethoxyliertes Glyzerin oder Trimethylolpropan, wie beispielsweise Polyol TP 70® (Perstorp, SE). Letztere haben insbesondere den Vorteil, dass sie die Oberflächenspannung eines wässrigen Extrakts der wasserabsorbierenden Polymerpartikel nur unwesentlich herabsetzen.

Es ist ebenso möglich, die erfindungsgemäßen Superabsorber durch Wasserzusatz auf einen gewünschten Wassergehalt einzustellen.

Wahlweise wird der erfindungsgemäße Superabsorber, der oberflächennachvernetzt ist, wobei die Nachvernetzungsreaktion bei einem Partialdruck oxidierender Gase von weniger als 140 mbar durchgeführt wird, mit weiteren Zusätzen versehen, die gegen Verfärbung stabilisieren und der erfindungsgemäße Superabsorber, der weiterhin Phosphinat und/oder Phosphonat enthält, wird mit diesen Zusätzen versehen. Beispiele sind insbesondere bekannte Stabilisatoren gegen Verfärbung, insbesondere reduzierende Substanzen. Unter diesen sind fest oder gelöste Salze der Phosphinsäure (H3PO2) sowie diese selbst bevorzugt. Beispielsweise eignen sich alle Phosphinate der Alkalimetalle, inklusive des Ammoniums, und der Erdalkalimetalle. Besonders bevorzugt sind wässrige Lösungen der Phosphinsäure welche Phosphinationen sowie mindestens ein Kation ausgewählt aus Natrium, Kalium, Ammonium, Calcium, Strontium, Aluminium, Magnesium enthalten. Ebenso bevorzugt sind Salze der Phosphonsäure (H3PO3) sowie diese selbst. Beispielsweise eignen sich alle primären und sekundären Phosphonate der Alkalimetalle, inklusive des Ammoniums, und der Erdalkalimetalle. Besonders bevorzugt sind wässrige Lösungen der Phosphonsäure, welche primäre und/oder sekundäre Phosphonationen sowie mindestens ein Kation ausgewählt aus Natrium, Kalium, Calcium, Strontium enthalten.

Alle Beschichtungen, Feststoffe, Zusätze und Hilfsstoffe können jeweils in separaten Verfahrensschritten zugegeben werden, meist ist jedoch die bequemste Methode, sie - falls sie nicht während der Versetzung des Grundpolymers mit Oberflächennachvernetzungsmittel zugegeben werden - dem Superabsorber im Kühler zuzugeben, etwa durch Aufsprühen einer Lösung oder Zugabe in feinteiliger fester oder in flüssiger Form.

Der L-Wert des Superabsorbers (CIE-Farbzahl) beträgt im nicht gelagerten Zustand typischerweise mindestens 75, vorzugsweise mindestens 80, besonders bevorzugt mindestens 85, ganz besonders bevorzugt mindestens 90, und höchstens 100.

Der a-Wert des Superabsorbers (CIE-Farbzahl) beträgt im nicht gelagerten Zustand typischerweise von -2,5 bis +2,5, vorzugsweise von -2,0 bis +2,0, besonders bevorzugt von -1,5 bis +1,5.

Der b-Wert des Superabsorbers (CIE-Farbzahl) beträgt im nicht gelagerten Zustand typischerweise von 0 bis 12, vorzugsweise von 2 bis 11.

Nach Lagerung bei erhöhter Temperatur und hoher Luftfeuchte weisen die erfindungsgemäßen Superabsorber nach Messung für die L- und a-Werte Ergebnisse im Bereich der Proben im nicht-gelagerten Zustand auf, und nach 100 Stunden Lagerung noch b-Werte von vorzugsweise nicht mehr als 12, besonders bevorzugt nicht mehr als 10, auf sowie nach 300 Stunden Lagerung noch b-Werte von vorzugsweise nicht mehr als 15, besonders bevorzugt nicht mehr als 12. Ein b-Wert oberhalb 12 ist in Damenhygieneartikeln und ultradünnen Windeln kritisch; ein b-Wert von mehr als 15 ist auch bereits in üblichen Windeln kritisch da diese Verfärbung vom Verbraucher bei Benutzung wahrgenommen werden kann.

Weiterhin sind die erfindungsgemäßen Superabsorber weitgehend frei von Verbindungen, die insbesondere während des Gebrauchs, zu unangenehmen Gerüchen führen.

In einer bevorzugten Ausführungsform der vorliegenden Erfindung wird das Sulfinsäure-Derivat in einem der Oberflächennachvernetzung nachgeschalteten Kühler und/oder in einem separaten nachgeschalteten Mischer auf die Oberfläche der nachvernetzten Polymerpartikel aufgebracht und das Initiatorsystem zur Herstellung des Polymeren

enthält ein Peroxodisulfat oder Peroxodiphosphat sowie mindestens eines der erfindungsgemässen Salze der 2-Hydroxy-2-sulfinatoessigsäure und der 2-Hydroxy-2-sulfonatoessigsäure oder deren freie Säuren, und wahlweise weitere Coinitiatoren.

In einer weiteren bevorzugten Ausführungsform der Erfindung werden Nachvernetzer (vorzugsweise 2-Oxazolidon oder N-(2-Hydroxyethyl)-2-oxazolidon und/oder 1,3-Propandiol), organisches Lösemittel und/oder Cosolvens (vorzugsweise Isopropanol und/oder 1,2-Propandiol), und wahlweise ein Tensid (bevorzugt Sorbitanmonolaurat, von vielen Herstellern als "Span® 20" (Marke der ICI Americas Inc., Wilmington, Delaware, U.S.A. erhältlich) mit Wasser gelöst und dann mittels eines Sprüh-Vertikalmischers (vorzugsweise ein Schugi®-Flexomix®) auf die Polymerpartikel mittels einer Zweistoffdüse oder Einstoffdüse aufgebracht, wobei sowohl der Mischer als auch ein direkt nachgeschalteter Trockner so mit Inertgas (vorzugsweise Stickstoff) gespült werden, dass der Volumenanteil des Sauerstoff in diesen Aggregaten weniger als 14 Vol.-%, vorzugsweise weniger als 10 Vol.-%, besonders bevorzugt weniger als 5 Vol.-%, ganz besonders bevorzugt weniger als 1 Vol.-%, beträgt. Über eine separate Zuführung wird zeitgleich zu dieser Nachvernetzungslösung eine Lösung mindestens eines Sulfinsäure-Derivats der Formel (I) und wahlweise eines mehrwertigen Metallsalzes und/oder eines weiteren Additivs aufgesprüht. Alternativ kann das Sulfinsäure-Derivat auch in der Nachvernetzungslösung oder einer ihrer Komponenten gelöst und mit der Nachvernetzungslösung gemeinsam aufgesprüht werden.

In einer weiteren bevorzugten Ausführungsform der Erfindung werden Nachvernetzer (vorzugsweise 2-Oxazolidon oder N-(2-Hydroxyethyl)-2-oxazolidon und/oder 1,3-Propandiol), organisches Lösemittel (vorzugsweise Isopropanol), Aluminiumlaktat, wahlweise ein weiteres Calciumsalz und wahlweise ein Tensid (bevorzugt Sorbitanmonolaurat) mit Wasser gelöst und dann mittels eines Sprüh-Vertikalmischers (vorzugs-weise ein Schugi®-Flexomix®) auf die Polymerpartikel mittels einer Zweistoffdüse oder Einstoffdüse aufgebracht, wobei sowohl der Mischer als auch ein direkt nachgeschalteter Trockner so mit Inertgas (vorzugsweise Stickstoff) gespült werden, dass der Volumenanteil Sauerstoff in diesen Aggregaten weniger als 14 Vol.-%, vorzugsweise weniger als 10 Vol.-%, besonders bevorzugt weniger als 5 Vol.-%, ganz besonders bevorzugt weniger als 1 Vol.-%, beträgt. Über eine separate Zuführung wird zeitgleich zu dieser Nachvernetzungslösung eine Lösung mindestens eines Sulfinsäure-Derivats der Formel (I) und wahlweise eines mehrwertigen Metallsalzes oder eines weiteren Additivs aufgesprüht. Alternativ kann das Sulfinsäure-Derivat auch in der Nachvernetzungslösung oder einer ihrer Komponenten gelöst und mit der Nachvernetzungslösung gemeinsam aufgesprüht werden.

In einer weiteren bevorzugten Ausführungsform der Erfindung werden Nachvernetzer (vorzugsweise Ethylengykoldigylcidylether oder 2-Oxazolidon oder N-(2-Hydroxyethyl)-2-oxazolidon und/oder 1,3-Propandiol und /oder 1,2-Propandiol), gegebenenfalls organisches Lösemittel (vorzugsweise Isopropanol), und wahlweise ein Tensid (bevorzugt Sorbitanmonolaurat) mit Wasser gelöst und dann mittels eines Sprüh-Vertikalmischers (vorzugsweise ein Schugi®-Flexomix®) auf die Polymerpartikel mittels einer Zweistoffdüse oder Einstoffdüse aufgebracht, wobei sowohl der Mischer als auch ein direkt nachgeschalteter Trockner so mit Inertgas (vorzugsweise Stickstoff) gespült werden, dass der Volumenanteil Sauerstoff in diesen Aggregaten weniger als 14 Vol.-%, vorzugsweise weniger als 10 Vol.-%, besonders bevorzugt weniger als 5 Vol.-%, ganz besonders bevorzugt weniger als 1 Vol.-%, beträgt. Während oder nach der Abkühlung des nachvernetzten Polymers wird in dieser bevorzugten Ausführungsform mindestens ein Sulfinsäure-Derivat, bevorzugt in wässriger Lösung, auf die Polymerpartikel aufgebracht.

In noch einer weiteren Ausführungsform der Erfindung werden Nachvernetzer (vorzugsweise ausgewählt aus Ethylengykoldigylcidylether, Ethylencarbonat, β-Hydroxialkylamiden, Polyolen, 2-Oxazolidon oder N-(2-Hydroxyethyl)-2-oxazolidon und/oder 1,3-Propandiol und /oder 1,2-Propandiol), gegebenenfalls organisches Lösemittel, und gegebenenfalls noch etwas Tensid (bevorzugt Sorbitanmonolaurat) mit Wasser gelöst und dann mittels eines Sprüh-Vertikalmischers (vorzugsweise ein Schugi®-Flexomix®) auf die Polymerpartikel mittels einer Zweistoffdüse oder Einstoffdüse aufgebracht, wobei sowohl der Mischer als auch ein direkt nachgeschalteter Trockner nicht mit Inertgas gespült werden. Während oder nach der Abkühlung des nachvernetzten Polymers wird in dieser Ausführungsform mindestens ein Sulfinsäure-Derivat der Formel (I) auf die Polymerpartikel aufgebracht. Bei dieser Ausführungsform wird wahlweise zusätzlich mindestens ein Sulfinsäure-Derivats der Formel (I) auf die Polymerpartikel vor oder während der Nachvernetzung aufgetragen, um die durch den atmosphärischen Sauerstoff hervorgerufene Vergilbung zu verringern.

Ein weiterer Gegenstand der vorliegenden Erfindung sind Hygieneartikel, enthaltend erfindungsgemäße Superabsorber, vorzugsweise ultradünne Windeln, enthaltend eine absorbierende Schicht bestehend aus 50 bis 100 Gew.-%, vorzugsweise 60 bis 100 Gew.-%, bevorzugt 70 bis 100 Gew.-%, besonders bevorzugt 80 bis 100 Gew.-%, ganz besonders bevorzugt 90 bis 100 Gew.-%, erfindungsgemäßer Superabsorber, wobei die Umhüllung der absorbierenden Schicht selbstverständlich nicht berücksichtigt ist.

Ganz besonders vorteilhaft sind die erfindungsgemäßen Superabsorber auch zur Herstellung von Laminaten und Kompositstrukturen, wie sie beispielsweise in der US 2003/0181115 sowie der US 2004/0019342 beschrieben sind, geeignet. Zusätzlich zu den in beiden Schriften zur Herstellung solcher neuer absorbierenden Strukturen beschriebenen Schmelzklebern und insbesondere den in der US 2003/0181115 beschriebenen Fasern aus Schmelzklebern, an die die Superabsorberpartikel gebunden ist, eignen sich die erfindungsgemäßen Superabsorber auch zur Herstellung von vollkommen analogen Strukturen unter Verwendung von UV-vernetzbaren Schmelzklebern, welche beispielsweise als AC-Resin® (BASF SE, Deutschland) vertrieben werden. Diese UV-vernetzbaren Schmelzkleber haben den Vorteil bereits bei 120 bis 140 °C verarbeitbar zu sein, daher sind sie mit vielen thermoplastischen Substraten besser kompatibel. Ein weiterer wesentlicher Vorteil besteht darin, dass UV-vernetzbare Schmelzkleber toxikologisch sehr unbedenklich sind und auch keine Ausdünstungen in den Hygieneartikeln verursachen. Ein ganz wesentlicher Vorteil, im Zusammenhang mit den erfindungsgemäßen Superabsorbern, ist die Eigenschaft der UV-vernetzbaren Schmelzkleber während der Verarbeitung und Vernetzung nicht zur Vergilbung zu neigen. Dies ist insbesondere von Vorteil, wenn ultradünne oder teilweise transparente Hygieneartikel hergestellt werden sollen. Die Kombination der erfindungsgemäßen Superabsorber mit UV-vernetzbaren Schmelzklebern ist daher besonders vorteilhaft. Geeignete UV-vernetzbare Schmelzkleber sind beispielsweise beschrieben in EP 0 377 199 A2, EP 0 445 641 A1, US 5,026,806, EP 0 655 465 A1 und EP 0 377 191 A2.

Der erfindungsgemäße Superabsorber kann außerdem in anderen Gebieten der Technik eingesetzt werden, bei denen Flüssigkeiten, insbesondere Wasser oder wässrige Lösungen absorbiert werden. Diese Gebiete sind beispielsweise Lagerung, Verpackung, Transport (als Bestandteile von Verpackungsmaterial für wasser- oder feuchtigkeitsempfindliche Artikel, etwa zum Blumentransport, auch als Schutz gegen mechanische Einwirkungen); Tierhygiene (in Katzenstreu); Lebensmittelverpackung (Transport von Fisch, Frischfleisch; Absorption von Wasser, Blut in Frischfisch- oder - fleischverpackungen); Medizin (Wundpflaster, wasserabsorbierendes Material für Brandverbände oder für andere nässende Wunden), Kosmetik (Trägermaterial für Pharmachemikalien und Medikamente, Rheumapflaster, Ultraschallgel, Kühlgel, Kosmetikverdicker, Sonnenschutz); Verdicker für Öl/Wasser bzw. Wasser/Öl-Emulsionen; Textilien (Feuchtigkeitsregulation in Textilien, Schuheinlagen, zur Verdampfungskühlung, etwa in Schutzkleidung, Handschuhen, Stirnbändern); chemisch-technische Anwendungen (als Katalysator für org. Reaktionen, zur Immobilisierung großer funktioneller Moleküle wie Enzymen, als Adhäsionsmittel bei Agglomerationen, Wärmespeicher, Filtrationshilfsmittel, hydrophile Komponente in Polymerlaminaten, Dispergiermittel, Verflüssiger); als Hilfsmittel beim Pulverspritzguss, im Bau- und Konstruktionswesen (Installation, in lehmbasierenden Putzen, als vibrationshemmendes Medium, Hilfsmittel bei Tunnelgrabungen in wasserreichem Untergrund, Kabelummantelung); Wasserbehandlung, Abfallbehandlung, Wasserabtrennung (Enteisungsmittel, wiederverwendbare Sandsäcke); Reinigung; Agrarindustrie (Bewässerung, Rückhaltung von Schmelzwasser und Tauniederschläge, Kompostierungszusatz, Schutz der Wälder vor Pilz-/Insektenbefall, verzögerte Freisetzung von Wirkstoffen an Pflanzen); zur Brandbekämpfung oder zum Brandschutz; Coextrusionsmittel in thermoplastischen Polymeren (z. B. zur Hydrophilierung von Mehrschichtfolien); Herstellung von Folien und thermoplastischen Formkörpern, die Wasser absorbieren können (z.B. Regen- und Tauwasser speichernde Folien für die Landwirtschaft; Superabsorber enthaltende Folien zum Frischhalten von Obst und Gemüse, die in feuchte Folien verpackt werden; Superabsorber-Polystyrol Coextrudate, beispielsweise für Lebensmittelverpackungen wie Fleisch, Fisch, Geflügel, Obst und Gemüse); oder als Trägersubstanz in Wirkstoffformulierungen (Pharma, Pflanzenschutz).

Die erfindungsgemäßen Artikel zur Absorption von Flüssigkeit unterscheiden sich von bekannten dadurch, dass sie den erfindungsgemäßen Superabsorber enthalten.

Es wurde außerdem ein Verfahren zur Herstellung von Artikeln zur Absorption von Flüssigkeit, insbesondere Hygieneartikeln gefunden, das dadurch gekennzeichnet ist, dass man bei der Herstellung des betreffenden Artikels mindestens einen erfindungsgemäßen Superabsorber einsetzt. Im übrigen sind Verfahren zur Herstellung solcher Artikel unter Einsatz von Superabsorber bekannt.

## Testmethoden

Der Superabsorber wird mit den nachfolgend beschriebenen Testmethoden geprüft.

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (European Disposables and Nonwovens Association, Avenue Eugene Plasky, 157, 1030 Brussels, Belgium, www.edana.org) und INDA (Association of the Nonwoven Fabrics Industry, 1100 Crescent Green, Suite 115, Cary, North Carolina 27518, U.S.A., www.inda.org). Diese Veröffentlichung ist sowohl von EDANA als auch von INDA erhältlich.

Alle nachfolgend beschriebenen Messungen sollten, wenn nicht anders angegeben, bei einer Umgebungstemperatur von 23 $\pm$ 2 °C und einer relativen Luftfeuchte von 50 $\pm$ 10 % durchgeführt werden. Die Superabsorberpartikel werden vor der Messung gut durchmischt, wenn nicht anders angegeben.

### Zentrifugenretentionskapazität (CRC, Centrifuge Retention Capacity)

Die Zentrifugenretentionskapazität des Superabsorbers wird gemäß der Standard-Testmethode Nr. WSP 241.5-02 "Centrifuge retention capacity" bestimmt.

Absorption unter Druck (AUL0.3psi, "Absorbency Under Load of 0.3 psi")

Die Absorption unter einem Druck von 2068 Pa (0.3 psi) des Superabsorbers wird gemäß der Standard-Testmethode Nr. WSP 242.2-05 "Absorption under pressure" bestimmt.

Absorption unter Druck (AUL0.7psi, "Absorbency Under Load of 0.7 psi)

Die Absorption unter einem Druck von 4826 Pa (0.7 psi) des Superabsorbers wird analog der Standard-Testmethode Nr. WSP 242.2-05 "Absorption under pressure" bestimmt, wobei jedoch ein Gewicht mit 49 g/$cm^2$ (führt zu einem Druck von 0.7 psi) statt eines Gewichts mit 21 g/$cm^2$ (führt zu einem Druck von 0.3 psi) verwendet wird.

Flüssigkeitsweiterleitung (SFC, "Saline Flow Conductivity")

Die Flüssigkeitsweiterleitung einer vom Superabsorber durch Flüssigkeitsabsorption gebildeten gequollenen Gelschicht wird unter Druckbelastung von 0.3 psi (2068 Pa), wie in EP 0 640 330 A1 beschrieben, als Gel-Layer-Permeability einer gequollenen Gelschicht aus Superabsorberpartikeln bestimmt, wobei die in zuvor genannter Patentanmeldung auf Seite 19 und in Figur 8 beschriebene Apparatur dahingehend modifiziert ist, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.
Die Flüssigkeitsweiterleitung (SFC) wird wie folgt berechnet:

$$SFC\ [cm^3s/g] = (Fg(t{=}0)xL0)/(dxAxWP),$$

wobei Fg(t=0) der Durchfluß an NaCl-Lösung in g/s ist, der anhand einer linearen Regressionsanalyse der Daten Fg(t) der Durchflußbestimmungen durch Extrapolation gegen t=0 erhalten wird, L0 die Dicke der Gelschicht in cm, d die Dichte der NaCl-Lösung in g/$cm^3$, A die Fläche der Gelschicht in $cm^2$ und WP der hydrostatische Druck über der Gelschicht in dyn/$cm^2$ darstellt.

Feuchtegehalt des Hydrogels (Restfeuchte, Wassergehalt)

Der Wassergehalt der wasserabsorbierenden Polymerpartikel wird gemäß der Standard-Testmethode Nr. WSP 230.2-05 "Moisture content" bestimmt.

Mittlere Partikelgröße

Die mittlere Partikelgröße der Produktfraktion wird gemäß der Standard-Testmethode Nr. WSP 220.2-05 "Partikel size distribution" ermittelt.

CIE-Farbzahl (L a b)

Die Farbmessung wird entsprechend dem CIELAB-Verfahren (Hunterlab, Band 8, Jahrgang 1996, Heft 7, Seiten 1 bis 4) mit einem Colorimeter, Modell "LabScan XE S/N LX17309" (HunterLab, Reston, U.S.A.) durchgeführt. Dabei werden die Farben über die Koordinaten L, a und b eines dreidimensionalen Systems beschrieben. Dabei gibt L die Helligkeit an, wobei L = 0 schwarz und L = 100 weiß bedeutet. Die Werte für a und b geben die Position der Farbe auf den Farbachsen rot/grün bzw. gelb/blau an, wobei +a für rot, -a für grün, +b für gelb und -b für blau steht.
Die Farbmessung entspricht dem Dreibereichsverfahren nach DIN 5033-6.

Lagertest

Messung 1: Eine Glasschale mit 9 cm Innendurchmesser und 1,5 cm Höhe wird mit wasserabsorbierenden Polymerpartikeln überfüllt und dann mit einem Messer über den Rand glatt gestrichen und die CIE-Farbzahlen bestimmt.

Messung 2a: Eine Glasschale mit 9 cm Innendurchmesser und 1,5 cm Höhe wird mit 15 g Superabsorberpartikeln

gefüllt und diese dann mit einem Messer glatt gestrichen. Die Schale wird dann offen in einen auf 65 °C temperierten Klimaschrank bei konstanter relativer Luftfeuchte von 90% gestellt. Die Schale wird nach Ablauf von 7 Tagen herausgenommen, der Inhalt durch Rühren vermischt und geglättet. Nach Abkühlen auf Raumtemperatur und werden die CIE-Farbzahlen bestimmt.

Messung 2b: Eine Glasschale mit 9 cm Innendurchmesser und 1,5 cm Höhe wird mit 12 g Superabsorberpartikeln gefüllt, so dass der Boden homogen bedeckt ist. Die Schale wird dann mit einem Glasdeckel bedeckt in einen auf 70 °C temperierten Klimaschrank bei konstanter relativer Luftfeuchte von 75 % gestellt. Die Schale wird nach Ablauf von 6 Tagen herausgenommen, die Partikel werden auf eine glatte Fläche gestürzt und die CIE-Farbzahlen nach Abkühlen der Probe auf Raumtemperatur auf der nun obenliegenden Unterseite der Probe bestimmt.

Beispiele

Beispiel 1 (Vergleich):

In dem als Polymerisationsreaktor verwendeten Pflugschar®-Schaufeltrockner mit 5 l Volumen und Heizmantel (Hersteller: Gebr. Lödige Maschinenbau GmbH, Elsener-Straße 7 - 9, 33102 Paderborn, Deutschland; Typ VT 5R-MK) wurden 206,5 g Wasser, 271,6 g Acrylsäure (stabilisiert mit 0,02 Gew.-% Hydrochinonmonomethylether), 2115,6 g einer 37,3 Gew.-%igen Natriumacrylatlösung (100 Mol-% neutralisiert), die zuvor zwecks Entfernung von Hydrochinonmonomethylether über Aktivkohle filtriert wurde, sowie 3,5g Triacrylat dreifach ethxoylierten Glycerins vorgelegt und durch 20-minütiges Durchperlen von Stickstoff inertisiert. Die Welle des Reaktors wurde durchgehend mit 100 Umdrehungen pro Minute gedreht. Der Gehalt an Hydrochinomonomethylether, bezogen auf Acrylsäure plus Acrylat, dieses auf Acrylsäure umgerechnet, betrug ca. 0,0064 Gew.-%. Die Reaktionsmischung wurde von außen (kühlmitteldurchströmter Reaktormantel) so gekühlt, dass die nachfolgende Initiatorzugabe bei ca. 20 °C erfolgte. Schließlich wurden in den Reaktor unter Rühren noch eine Initiatormischung aus 0,4 g Natriumpersulfat (gelöst in 12 g Wasser) und 0,13 g Brüggolit® FF7 (gelöst in 10 g Wasser) hinzugefügt und die Kühlung abgestellt. Die Reaktion setzte zügig ein. Ab Erreichen einer Innentemperatur des Reaktors von 30 °C wurde der Mantel mit 80 °C heißem Wärmeträgermedium beheizt, um die Reaktion möglichst adiabat zu Ende zu führen. Nach Erreichen der Maximaltemperatur wurde wiederum gekühlt (Kühlflüssigkeit mit -12 °C), so das entstandene Gel auf unter 50°C herabgekühlt und dann ausgetragen.
Das entstandene Gel wurde auf zwei Bleche mit Drahtboden verteilt und bei 160 °C in einem Umlufttrockenschrank getrocknet. Anschließend wurde mit einer Ultrazentrifugalmühle zerkleinert und das Produkt auf eine Korngröße von 150 bis 710 μm abgesiebt. Das so hergestellte Grundpolymer hatte eine CRC von 35,8 g/g und eine AUL 0.3 psi von 17,5 g/g.
Das so hergestellte Grundpolymer wurde zur Oberflächennachvernetzung in einem Pflugschar®-Mischer mit Heizmantel (Hersteller: Gebr. Lödige Maschinenbau GmbH, Elsener-Straße 7 - 9, 33102 Paderborn, Deutschland; Typ M5) bei Raumtemperatur und einer Wellendrehzahl von 450 Umdrehungen pro Minute mittels zwei Zweistoff-Sprühdüsen mit folgenden Lösungen beschichtet:

| | |
|---|---|
| Lösung 1: | 0,1 Gew.-% Ethylenglykoldiglycidylether (Denacol® EX-810 von Nagase ChemteX Corporation, Osaka, Japan), bezogen auf Grundpolymer 0,60 Gew.-% 1,2-Propandiol bezogen auf Grundpolymer 0,75 Gew.-% Wasser bezogen auf Grundpolymer |
| Lösung 2: | 3,0 Gew.-% wässrige Aluminiumsulfat-Lösung (26,8 Gew.-%ig) bezogen auf Grundpolymer |

Nach dem Aufsprühen wurde die Produkttemperatur auf 170 °C erhöht und das Reaktionsgemisch 45 Minuten lang bei dieser Temperatur und einer Wellendrehzahl von 80 Umdrehungen pro Minute gehalten. Das erhaltene Produkt wurde wieder auf Raumtemperatur abkühlen gelassen und gesiebt. Das oberflächennachvernetzte Polymer (der Superabsorber) wurde als Siebfraktion mit Partikelgrößen zwischen 150 μm und 850 μm gewonnen.

Beispiel 2 (Vergleich)

Der im Beispiel 1 hergestellte Superabsorber wurde in einem Pflugschar®-Mischer mit Heizmantel (Hersteller: Gebr. Lödige Maschinenbau GmbH, Elsener-Straße 7 - 9, 33102 Paderborn, Deutschland; Typ M5) innerhalb von 20 Minuten bei einer Wellendrehzahl von 450 Umdrehungen pro Minute mit 0,10 Gew.% Sipernat® D17 homogen durchmischt und gleichzeitig mittels einer Zweistoff-Sprühdüse mit einer wässrigen Lösung von 0,08 Gew.% Polyethylenglykol-400 und 2,0 Gew.-% Wasser (jeweils auf eingesetzten Superabsorber bezogen) bei Raumtemperatur beschichtet.

Beispiel 3 (Vergleich)

Der im Beispiel 1 hergestellte Superabsorber wurde in einem Pflugschar®-Mischer mit Heizmantel (Hersteller: Gebr. Lödige Maschinenbau GmbH, Elsener-Straße 7 - 9, 33102 Paderborn, Deutschland; Typ M5) innerhalb von 20 Minuten bei einer Wellendrehzahl von 450 Umdrehungen pro Minute mit 0,10 Gew.-% Sipernat® D17 homogen durchmischt und gleichzeitig mittels einer Zweistoff-Sprühdüse mit einer wässrigen Lösung von 0,08 Gew.-% Polyethylenglykol-400 sowie 0,40 Gew.-% Natriumbisulfit und 2,0 Gew.-% Wasser (jeweils auf eingesetztes Grundpolymer bezogen) bei Raumtemperatur beschichtet.

Beispiel 4 (Vergleich)

Es wurde wie in Beispiel 3 verfahren, wobei jedoch 0,80 Gew.-% Natriumbisulfit eingesetzt wurden.

Beispiel 5 (Vergleich)

Es wurde wie in Beispiel 3 verfahren, wobei jedoch 1,20 Gew.-% Natriumbisulfit eingesetzt wurden.

Beispiel 6 (Vergleich):

Es wurde wie bei der Herstellung des Grundpolymers in Beispiel 1 verfahren, wobei jedoch 1,29 g Triacrylat dreifach ethoxilierten Glycerins, 0,618 g Natriumpersulfat und anstatt des Brüggolit® FF7 in 10 g Wasser 0,013 g Ascorbinsäure in 9,12 g Wasser eingesetzt wurden. Das erhaltene Produkt wurde ferner abschließend auf eine Korngröße von 200 bis 600 mm abgesiebt. Es wurde nicht oberflächennachvernetzt.

Beispiel 7 (Vergleich):

Ein Grundpolymer (üblicher, nicht oberflächennachvernetzter Polyacrylat-Superabsorber mit CRC = 36 g/g, AUL 0.3 psi = 16 g/g und einer Kornverteilung (Mittelwerte) < 150 $\mu$m = 0,5 Gew.%; > 150 $\mu$m = 15,8 Gew.%, > 300 $\mu$m = 70, 9 Gew.-%; > 600 $\mu$m = 12, 8 Gew.-% und > 710 $\mu$m = 0,05 Gew.%) wurde zur Oberflächennachvernetzung durch einen Mischer (Schugi® Flexomix® Typ 100 D, Hersteller: Hosokawa Micron B.V. Gildenstraat 26, 7005 BL Doetinchem, Niederlande) geführt. Die Dosierung des Grundpolymers erfolgte gravimetrisch, der Durchsatz betrug 80 kg/h. Die Welle wurde mit 3400 Umdrehungen pro Minute gedreht, die Paddel standen horizontal. Dabei wurden zeitgleich zwei Lösungen oder Dispersionen I und II in geregelten kontinuierlichen Massenfluss über je eine Zweistoffdüse auf das Polymer gesprüht:

Lösung I war ein Gemisch aus 0,2 Gew.-% Wasser, 0,8 Gew.-% wässrige Aluminiumlaktatlösung (eine 25 Gew.-%ige wässrige Lösung; Lohtragon® AL 250 von Dr. Paul Lohmann GmbH KG, Hauptstraße 2, 31860 Emmerthal, Deutschland, www.lohmann-chemikalien.de), 0,05 Gew.-% 2-Hydroxyethyloxazolidinon, 0,05 Gew.-% Propandiol-1,3, 0,5 Gew.-% Propylenglykol-1,2, 0,008 Gew.-% Sorbitanmonolaurat und 0,87 Gew.-% Isopropanol, jeweils auf das damit behandelte Grundpolymer bezogen. Lösung I wurde in einer Menge von 1,982 kg/h durch eine feine Flüssigkeitsdüse (Typ J-2850-SS + Gasdüse J-73328-SS von Spraying Systems Deutschland GmbH, Großmoorkehre 1, 21079 Hamburg, Deutschland) aufgesprüht, die auf der Höhe des Feststoffeinlaufs am Schugi® Flexomix® um 90° versetzt zu dessen zentraler Achse angeordnet war. Als Sprühgas wurde Stickstoff mit einem Vordruck von je 2 bar verwendet.

Lösung II (eigentlich eine Dispersion) bestand aus 0,875 Gew.-% Wasser, 0,4 Gew.-% Aluminiumlaktat-Lösung (eine 25 Gew.-%ige wässrige Lösung; Lohtragon® AL 250 von Dr. Paul Lohmann GmbH KG, Hauptstraße 2, 31860 Emmerthal, Deutschland, www.lohmann-chemikalien.de), 0,3 Gew.-% Tricalciumphosphat C53-80 (Chemische Fabrik Budenheim KG, Rheinstrasse 27, 55257 Budenheim, Deutschland) und 0,05 Gew.-% Brüggolit® FF7 (L. Brüggemann KG, Salzstraße 131, 74076 Heilbronn, Deutschland, www.brueggemann.com), jeweils auf das damit behandelte Grundpolymer bezogen. Das Brüggolit® FF7 wurde dabei zunächst in Wasser gelöst, die Aluminiumlaktat-Lösung zugegeben und dann das Tricalciumphosphat in dieser Lösung mit einem Hochgeschwindigkeitsrührer (Ultra-Turrax® T50 mit S50KR-Schaft, IKA® Werke GmbH & Co. KG, Janke & Kunkel-Str. 10, 79219 Staufen, Deutschland) dispergiert. Im Vorlagegefäß wurde die Suspension durch Rühren homogen gehalten. Lösung II wurde in einer Menge von 1,30 kg/h über eine gröbere Flüssigkeitsdüse (Typ J-60100-SS + Gasdüse J-125328-SS von Spraying Systems Deutschland GmbH, Großmoorkehre 1, 21079 Hamburg, Deutschland) aufgesprüht, die auf der Höhe des Feststoffeinlaufs am Schugi® Flexomix® um 270° versetzt zu dessen zentraler Achse angeordnet war. Als Sprühgas wurde Stickstoff mit einem Vordruck von je 2 bar verwendet.

Das feuchte Polymer wurde direkt aus dem Schugi® Flexomix®-Mischer fallend in einen mit Dampf beheizten Paddeltrockner (Typ NPD 1.6 W, bezogen von Nara Machinery Co., Ltd., Zweigniederlassung Europa, Europaallee 46, 50226 Frechen, Deutschland) überführt. Die Temperatur im Trockner wurde so eingestellt, dass ein Sollwert der Produkttemperatur am Trocknerausgang von 187°C erreicht wurde. Durch die Aufstellung des Trockners mit einer Neigung Richtung Austrag von 3°, einer Wehrhöhe von ca. 64 mm, was einem Füllgrad von ca. 95% entspricht und einer Umdrehung der Welle von ca. 14 UpM wurde eine mittlere Verweilzeit des Produktes im Trockner von ca. 35 Minuten eingestellt. Dem Trockner war ein Schneckenkühler mit 50 l Volumen (Hersteller: Lurgi Gesellschaft für Wärmetechnik mbH, Frankfurt/Main, Deutschland nachgeschaltet, in dem das Produkt auf ca. 50°C abgekühlt wurde. Anschließend wurde das Produkt über eine Siebmaschine geleitet, die mit 2 Siebdecks (150 mm / 710mm) ausgestattet war, wobei ca. 3 Gew.-% Polymer (bezogen auf eingesetztes Grundpolymer) überwiegend als Grobgut abgetrennt wurden. Der Superabsorber der Siebfraktion von 150-710 mm wurde als Gutprodukt gewonnen.

Beispiel 8: Superabsorber von Beispiel 7, nachbehandelt mit Brüggolit® FF7 (Vergleich)

Der Superabsorber aus Beispiel 7 wurde ein zweites Mal auf dieselbe Weise durch dieselbe, in Beispiel 7 beschriebene Vorrichtung zur Oberflächennachvernetzung geführt, wobei die gleichen Parameter eingestellt wurden, jedoch wurde im Schugi® Flexomix® nur eine Lösung aus 0,95 Gew.-% Wasser und 0,05 Gew.-% Brüggolit® FF7, bezogen auf das Gewicht des Superabsorbers, über die in der 90°-Stellung angeordnete Düse aufgesprüht und die Temperatur im Paddeltrockner so eingestellt, dass ein Sollwert der Produkttemperatur am Ausgang von 100°C erreicht wurde.

Beispiel 9: Superabsorber, mit Al-Laktat und Brüggolit® FF7 (Vergleich)

Beispiel 7 wurde wiederholt, dabei wurde jedoch im Schugi® Flexomix® nur eine Lösung zu gleichen Teilen über beide Düsen aufgesprüht, wobei zudem identische Düsen verwendet wurden (Typ J-2850-SS + Gasdüse J-73328-SS). Die Zusammensetzung der aufgesprühten Lösung war: 0,76 Gew.-% Wasser, 0,075 Gew.-% Brüggolit® FF7, 1,8 Gew.-% Aluminiumlaktatlösung (25%ige wässrige Lösung; Lohtragon® AL 250), 0,05 Gew.-% 2-Hydroxyethyloxazolidinon, 0,5 Gew.-% Propylenglykol-1,2, 0,008 Gew.-% Sorbitanmonolaurat und 0,91 Gew.-% Isopropanol, jeweils auf das eingesetzte Grundpolymer bezogen.

Beispiel 10 (Vergleich):

Beispiel 7 wurde wiederholt, wobei jedoch während der Oberflächennachvernetzung kein Brüggolit® FF7 zugesetzt wurde.

Beispiel 11 (Vergleich):

Auf 1200 g des im Beispiel 10 hergestellten Superabsorbers wurde in einem Pflugschar®-Mischer mit Heizmantel (Hersteller: Gebr. Lödige Maschinenbau GmbH, Elsener-Straße 7 - 9, 33102 Paderborn, Deutschland; Typ M5) innerhalb von 2 Minuten bei einer Wellendrehzahl von 450 Umdrehungen pro Minute mittels einer Zweistoff-Sprühdüse eine Lösung aus 40,8 g Wasser, 0,12 g Brüggolit® FF7 und 0,72 g Ca-Laktat bei Raumtemperatur gesprüht. Anschließend wurde das erhaltene Polymer für 60 Minuten bei 100°C in einem Umlufttrockenschrank getrocknet. Mittels eines Siebes mit 850 μm Maschenweite wurden Grobanteile entfernt.

Die Superabsorber der Beispiele 1 bis 11 wurden dem Lagertest unterworfen. Farbzahlen wurden dabei nach Messung 1a und Messung 2b erhalten. Die Ergebnisse sind in der nachfolgenden Tabelle zusammengestellt:
Die Absorptionseigenschaften der Superabsorber aus Beispielen 1 - 11 waren wie folgt:

| Beispiel | CRC [g/g] | AUL 0.7 psi [g/g] | SFC [$10^{-7}$ $cm^3s/g$] |
|---|---|---|---|
| 1 (Vergleich) | 32,9 | 20,6 | 22 |
| 2 (Vergleich) | 32.6 | 20.2 | 26 |
| 3 (Vergleich) | 32.5 | 19.8 | 28 |
| 4 (Vergleich) | 32.8 | 20.0 | 24 |
| 5 (Vergleich) | 32.4 | 19.7 | 21 |

(fortgesetzt)

| Beispiel | CRC [g/g] | AUL 0.7 psi [g/g] | SFC [$10^{-7}$ $cm^3s/g$] |
|---|---|---|---|
| 6 (Vergleich) | 35,6 | < 10 g/g | nicht bestimmt |
| 7 (Vergleich) | 28,1 | 24,3 | 130 |
| 8 (Vergleich) | 29,0 | 24,4 | 100 |
| 9 (Vergleich) | 28,8 | 25,4 | 117 |
| 10 (Vergleich) | 28,4 | 24,2 | 109 |
| 11 (Vergleich) | 29,2 | 24,3 | 99 |

Die Superabsorber der Beispiele 1 - 11 wurden dem Lagertest unterworfen. Die Ergebnisse sind in der nachfolgenden Tabelle zusammengestellt:

| Beispiel | vor Lagerung, Messung 1 | | | nach Lagerung, Messung 2a (Bsp. 2-5) oder 2b (Bsp. 1, 6-11) | | |
|---|---|---|---|---|---|---|
| | L | a | b | L | a | b |
| 1 (Vergleich) | 91,12 | -0,85 | 9,66 | 87,16 | -0,18 | 9,18 |
| 2 (Vergleich) | 89,4 | -1,1 | 3,9 | 79,2 | 1,5 | 6,9 |
| 3 (Vergleich) | 89,0 | -1,6 | 6,5 | 83,8 | 0,1 | 4,1 |
| 4 (Vergleich) | 88,4 | -1,6 | 7,7 | 83,8 | 0,0 | 4,7 |
| 5 (Vergleich) | 89,9 | -1,7 | 7,3 | 84,3 | 0,1 | 4,5 |
| 6 (Vergleich) | 88,76 | -0,42 | 11,35 | 76,49 | 3,48 | 13,84 |
| 7 (Vergleich) | 90,96 | -1,07 | 10,4 | 84,69 | 2,42 | 8,49 |
| 8 (Vergleich) | 90,59 | -1,05 | 10,47 | 86,38 | 0,30 | 11,51 |
| 9 (Vergleich) | 92,19 | -1,11 | 9,78 | 90,73 | 0,33 | 9,56 |
| 10 (Vergleich) | 92,75 | -0,85 | 9,78 | 81,53 | 3,38 | 11,59 |
| 11 (Vergleich) | 91,82 | -1,03 | 9,46 | 87,86 | -0,13 | 9,58 |

Der Vergleich der Messwerte der Beispiele 2-5 zeigt eine deutliche Verfärbung der Proben bei Lagerung, wobei die nach dem Stand der Technik mit Natriumhydrogensulfit behandelten Proben signifikant gegen diese Verfärbung stabilisiert sind, beim vergleichsweise hohen Hydrogensulfitgehalt von Beispiel 5 der SFC-Wert aber etwas abnimmt.

Ein Vergleich von Beispielen 6 (ohne Sulfinat-Initiator) und 1 (mit Sulfinat-Initiator) zeigt, dass bereits die Verwendung von Sulfinsäuresalz als Teil des Redoxinititators nach dem Stand der Technik eine Verbesserung der Farbstabilität bewirkt, obwohl nach der Polymerisation kein Initiator mehr im Produkt enthalten ist. Der erfindungsgemäße Zusatz eines Sulfinsäure-Derivats nach der Polymerisation zeigt jedoch eine nochmals verbesserte Farbstabilität.

**Patentansprüche**

1. Superabsorber, enthaltend eine Verbindung der Formel (I)

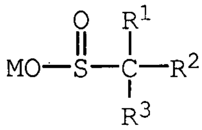

(I),

worin

M für ein Wasserstoffatom, ein Ammoniumion, ein einwertiges Metallion oder ein Äquivalent eines zweiwertigen Metallions der Gruppen 1, 2, 8, 9, 10, 12 oder 14 des Periodensystems der Elemente steht;
$R^1$ für OH oder $NR^4R^5$ steht, wobei $R^4$ und $R^5$ unabhängig voneinander für H oder $C_1$-$C_6$-Alkyl stehen;
$R^2$ für H oder eine Alkyl-, Alkenyl-, Cycloalkyl- oder Arylgruppe steht, wobei diese Gruppe wahlweise 1, 2 oder 3 Substituenten aufweist, die unabhängig voneinander ausgewählt sind unter $C_1$-$C_6$-Alkyl, OH, O-$C_1$-$C_6$-Alkyl, Halogen und $CF_3$; und
$R^3$ für COOM, $SO_3M$, $COR^4$, $CONR^4R^5$ oder $COOR^4$ steht, wobei M, $R^4$ und $R^5$ die oben angegebenen Bedeutungen besitzen oder, wenn $R^2$ für Aryl steht, das wahlweise wie oben angegeben substituiert ist, auch für H steht,

Salzen davon oder Gemischen solcher Verbindungen und/oder Salzen davon, **dadurch gekennzeichnet, dass** er oberflächennachvernetzt ist, wobei die Nachvernetzungsreaktion bei einem Partialdruck oxidierender Gase von weniger als 140 mbar durchgeführt wurde.

**2.** Superabsorber nach Anspruch 1, **dadurch gekennzeichnet, dass** M für ein Alkalimetallion oder ein Äquivalent eines Erdalkalimetall- oder Zinkions steht; $R^1$ für eine Hydroxi- oder Aminogruppe steht; $R^2$ für H oder Alkyl steht und $R^3$ für COOM oder $COOR^4$ steht, wobei, wenn $R^3$ für COOM steht, M in diesem COOM-Rest für H, ein Alkalimetallion oder ein Äquivalent eines Erdalkalimetallions steht und wenn $R^3$ für $COOR^4$ steht, $R^4$ für $C_1$-$C_6$-Alkyl steht.

**3.** Superabsorber nach Anspruch 1, **dadurch gekennzeichnet, dass** M für ein Alkalimetallion oder ein Äquivalent eines Erdalkalimetall- oder Zinkions steht; $R^1$ für eine Hydroxi- oder Aminogruppe steht; $R^2$ für Aryl, das wahlweise substituiert ist, steht und $R^3$ für ein Wasserstoffatom steht.

**4.** Superabsorber nach Anspruch 1, **dadurch gekennzeichnet, dass** er ein Gemisch aus dem Natriumsalz der 2-Hydroxy-2-sulfinatoessigsäure, dem Dinatriumsalz der 2-Hydroxy-2-sulfonatoessigsäure und Natriumbisulfit enthält.

**5.** Superabsorber nach einem der Ansprüche 1 bis 4, **dadurch gekennzeichnet, dass** er mindestens 0,0001 Gew.-% und höchstens 3 Gew.-% Verbindung der Formel (I), Salzen davon oder Gemischen solcher Verbindungen und/oder Salzen davon enthält, jeweils auf das Gesamtgewicht des erfindungsgemäßen Superabsorbers bezogen.

**6.** Verfahren zur Herstellung eines in den Ansprüchen 1 bis 5 definierten Superabsorbers, umfassend das Vermischen einer Verbindung der Formel (I), Salzen davon oder Gemischen solcher Verbindungen und/oder Salzen davon mit einem Superabsorber und die Oberflächennachvernetzung des Superabsorbers, wobei die Nachvernetzungsreaktion bei einem Partialdruck oxidierender Gase von weniger als 140 mbar durchgeführt wird.

**7.** Artikel zur Absorption von Flüssigkeiten, enthaltend einen der in Ansprüchen 1 bis 5 definierten Superabsorber.

**8.** Verfahren zur Herstellung von Artikeln zur Absorption von Flüssigkeit, **dadurch gekennzeichnet, dass** bei der Herstellung einer der in Ansprüchen 1 bis 5 definierten Superabsorber eingesetzt wird.

**9.** Superabsorber, enthaltend eine Verbindung der Formel (I)

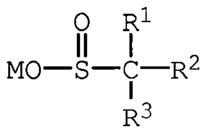

(I),

worin

M für ein Wasserstoffatom, ein Ammoniumion, ein einwertiges Metallion oder ein Äquivalent eines zweiwertigen Metallions der Gruppen 1, 2, 8, 9, 10, 12 oder 14 des Periodensystems der Elemente steht;
$R^1$ für OH oder $NR^4R^5$ steht, wobei $R^4$ und $R^5$ unabhängig voneinander für H oder $C_1$-$C_6$-Alkyl stehen;
$R^2$ für H oder eine Alkyl-, Alkenyl-, Cycloalkyl- oder Arylgruppe steht, wobei diese Gruppe wahlweise 1, 2 oder 3 Substituenten aufweist, die unabhängig voneinander ausgewählt sind unter $C_1$-$C_6$-Alkyl, OH, O-$C_1$-$C_6$-Alkyl, Halogen und $CF_3$; und
$R^3$ für COOM, $SO_3M$, $COR^4$, $CONR^4R^5$ oder $COOR^4$ steht, wobei M, $R^4$ und $R^5$ die oben angegebenen Bedeutungen besitzen oder, wenn $R^2$ für Aryl steht, das wahlweise wie oben angegeben substituiert ist, auch für

H steht,

Salzen davon oder Gemischen solcher Verbindungen und/oder Salzen davon, **dadurch gekennzeichnet, dass** er weiterhin Phosphinat und/oder Phosphonat enthält.

**10.** Superabsorber nach Anspruch 9, **dadurch gekennzeichnet, dass** M für ein Alkalimetallion oder ein Äquivalent eines Erdalkalimetall- oder Zinkions steht; $R^1$ für eine Hydroxi- oder Aminogruppe steht; $R^2$ für H oder Alkyl steht und $R^3$ für COOM oder $COOR^4$ steht, wobei, wenn $R^3$ für COOM steht, M in diesem COOM-Rest für H, ein Alkalimetallion oder ein Äquivalent eines Erdalkalimetallions steht und wenn $R^3$ für $COOR^4$ steht, $R^4$ für $C_1$-$C_6$-Alkyl steht.

**11.** Superabsorber nach Anspruch 9, **dadurch gekennzeichnet, dass** M für ein Alkalimetallion oder ein Äquivalent eines Erdalkalimetall- oder Zinkions steht; $R^1$ für eine Hydroxi- oder Aminogruppe steht; $R^2$ für Aryl, das wahlweise substituiert ist, steht und $R^3$ für ein Wasserstoffatom steht.

**12.** Superabsorber nach Anspruch 9, **dadurch gekennzeichnet, dass** er ein Gemisch aus dem Natriumsalz der 2-Hydroxy-2-sulfinatoessigsäure, dem Dinatriumsalz der 2-Hydroxy-2-sulfonatoessigsäure und Natriumbisulfit enthält.

**13.** Superabsorber nach einem der Ansprüche 9 bis 12, **dadurch gekennzeichnet, dass** er mindestens 0,0001 Gew.-% und höchstens 3 Gew.-% Verbindung der Formel (I), Salzen davon oder Gemischen solcher Verbindungen und/oder Salzen davon enthält, jeweils auf das Gesamtgewicht des erfindungsgemäßen Superabsorbers bezogen.

**14.** Superabsorber nach einem der Ansprüche 9 bis 13, **dadurch gekennzeichnet, dass** er oberflächennachvernetzt ist.

**15.** Verfahren zur Herstellung eines in den Ansprüchen 9 bis 14 definierten Superabsorbers, umfassend das Vermischen einer Verbindung der Formel (I), Salzen davon oder Gemischen solcher Verbindungen und/oder Salzen davon sowie eines Phosphinats und/oder Phosphonats mit einem Superabsorber.

**16.** Artikel zur Absorption von Flüssigkeiten, enthaltend einen der in Ansprüchen 9 bis 14 definierten Superabsorber.

**17.** Verfahren zur Herstellung von Artikeln zur Absorption von Flüssigkeit, **dadurch gekennzeichnet, dass** bei der Herstellung einer der in Ansprüchen 9 bis 14 definierten Superabsorber eingesetzt wird.

## Claims

**1.** A superabsorbent comprising a compound of the formula (I)

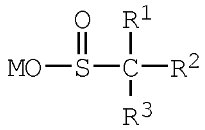

(I)

in which

M is a hydrogen atom, an ammonium ion, a monovalent metal ion or one equivalent of a divalent metal ion of groups 1, 2, 8, 9, 10, 12 or 14 of the periodic table of the elements;
$R^1$ is OH or $NR^4R^5$ where $R^4$ and $R^5$ are each independently H or $C_1$-$C_6$-alkyl;
$R^2$ is H or an alkyl, alkenyl, cycloalkyl or aryl group, where this group optionally has 1, 2 or 3 substituents which are each independently selected from $C_1$-$C_6$-alkyl, OH, O-$C_1$-$C_6$-alkyl, halogen and $CF_3$; and
$R^3$ is COOM, $SO_3M$, $COR^4$, $CONR^4R^5$ or $COOR^4$, where M, $R^4$ and $R^5$ are each as defined above or, when $R^2$ is aryl which is optionally substituted as specified above, are also H,

salts thereof or mixtures of such compounds and/or salts thereof, wherein it is surface postcrosslinked, the postcrosslinking reaction having been carried out at a partial pressure of oxidizing gases of less than 140 mbar.

**2.** The superabsorbent according to claim 1, wherein M is an alkali metal ion or one equivalent of an alkaline earth metal or zinc ion; $R^1$ is a hydroxyl or amino group; $R^2$ is H or alkyl and $R^3$ is COOM or $COOR^4$, where, when $R^3$ is

COOM, M in this COOM radical is H, an alkali metal ion or one equivalent of an alkaline earth metal ion, and, when $R^3$ is $COOR^4$, $R^4$ is $C_1$-$C_6$-alkyl.

**3.** The superabsorbent according to claim 1, wherein M is an alkali metal ion or one equivalent of an alkaline earth metal or zinc ion; $R^1$ is a hydroxyl or amino group; $R^2$ is aryl which is optionally substituted, and $R^3$ is a hydrogen atom.

**4.** The superabsorbent according to claim 1, which comprises a mixture of the sodium salt of 2-hydroxy-2-sulfinatoacetic acid, the disodium salt of 2-hydroxy-2-sulfonatoacetic acid and sodium bisulfite.

**5.** The superabsorbent according to any one of claims 1 to 4, which comprises at least 0.0001% by weight and at most 3% by weight of compound of the formula (I), salts thereof or mixtures of such compounds and/or salts thereof, based in each case on the total weight of the inventive superabsorbent.

**6.** A process for producing a superabsorbent defined in claims 1 to 5, comprising the mixing of a compound of the formula (I), salts thereof or mixtures of such compounds and/or salts thereof with a superabsorbent and the surface postcrosslinking of the superabsorbent, the postcrosslinking reaction being carried out at a partial pressure of oxidizing gases of less than 140 mbar.

**7.** An article for absorbing fluids, comprising a superabsorbent defined in claims 1 to 5.

**8.** A process for producing articles for absorbing fluid, which comprises using a superabsorbent defined in claims 1 to 5 in the production.

**9.** A superabsorbent comprising a compound of the formula (I)

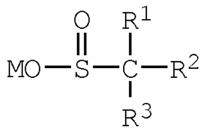

(I)

in which

M is a hydrogen atom, an ammonium ion, a monovalent metal ion or one equivalent of a divalent metal ion of groups 1, 2, 8, 9, 10, 12 or 14 of the periodic table of the elements;
$R^1$ is OH or $NR^4R^5$ where $R^4$ and $R^5$ are each independently H or $C_1$-$C_6$-alkyl;
$R^2$ is H or an alkyl, alkenyl, cycloalkyl or aryl group, where this group optionally has 1, 2 or 3 substituents which are each independently selected from $C_1$-$C_6$-alkyl, OH, O-$C_1$-$C_6$-alkyl, halogen and $CF_3$; and
$R^3$ is COOM, $SO_3M$, $COR^4$, $CONR^4R^5$ or $COOR^4$, where M, $R^4$ and $R^5$ are each as defined above or, when $R^2$ is aryl which is optionally substituted as specified above, are also H,

salts thereof or mixtures of such compounds and/or salts thereof,
which further comprises phosphinate and/or phoshonate.

**10.** The superabsorbent according to claim 9, wherein M is an alkali metal ion or one equivalent of an alkaline earth metal or zinc ion; $R^1$ is a hydroxyl or amino group; $R^2$ is H or alkyl and $R^3$ is COOM or $COOR^4$, where, when $R^3$ is COOM, M in this COOM radical is H, an alkali metal ion or one equivalent of an alkaline earth metal ion, and, when $R^3$ is $COOR^4$, $R^4$ is $C_1$-$C_6$-alkyl.

**11.** The superabsorbent according to claim 9, wherein M is an alkali metal ion or one equivalent of an alkaline earth metal or zinc ion; $R^1$ is a hydroxyl or amino group; $R^2$ is aryl which is optionally substituted, and $R^3$ is a hydrogen atom.

**12.** The superabsorbent according to claim 9, which comprises a mixture of the sodium salt of 2-hydroxy-2-sulfinatoacetic acid, the disodium salt of 2-hydroxy-2-sulfonatoacetic acid and sodium bisulfite.

**13.** The superabsorbent according to any one of claims 9 to 12, which comprises at least 0.0001% by weight and at most 3% by weight of compound of the formula (I), salts thereof or mixtures of such compounds and/or salts thereof, based in each case on the total weight of the inventive superabsorbent.

**14.** The superabsorbent according to any one of claims 9 to 13, which is surface postcrosslinked.

**15.** A process for producing a superabsorbent defined in claims 9 to 14, comprising the mixing of a compound of the formula (I), salts thereof or mixtures of such compounds and/or salts thereof, and a phosphinate and/or phosphonate, with a superabsorbent.

**16.** An article for absorbing fluids, comprising a superabsorbent defined in claims 9 to 14.

**17.** A process for producing articles for absorbing fluid, which comprises using a superabsorbent defined in claims 9 to 14 in the production.

## Revendications

**1.** Superabsorbant, contenant un composé de formule (I)

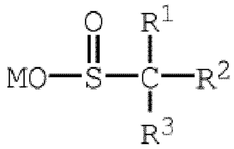

(I),

dans laquelle

M représente un atome d'hydrogène, un ion ammonium, un ion de métal monovalent ou un équivalent d'un ion de métal bivalent des groupes 1, 2, 8, 9, 10, 12 ou 14 du tableau périodique des éléments ;
$R^1$ représente OH ou $NR^4R^5$, $R^4$ et $R^5$ représentant indépendamment l'un de l'autre H ou alkyle en $C_1$-$C_6$ ;
$R^2$ représente H ou un groupe alkyle, alcényle, cycloalkyle ou aryle, ce groupe comprenant éventuellement 1, 2 ou 3 substituants, qui sont choisis indépendamment les uns des autres parmi alkyle en $C_1$-$C_6$, OH, O-alkyle en $C_1$-$C_6$, halogène et $CF_3$ ; et
$R^3$ représente COOM, $SO_3M$, $COR^4$, $CONR^4R^5$ ou $COOR^4$, M, $R^4$ et $R^5$ ayant les significations indiquées précédemment ou, lorsque $R^2$ représente aryle, qui est éventuellement substitué tel qu'indiqué précédemment, représente également H,
ses sels ou des mélanges de tels composés et/ou leurs sels, **caractérisé en ce qu'**il est post-réticulé en surface, la réaction de post-réticulation ayant été réalisée à une pression partielle de gaz oxydants inférieure à 140 mbars.

**2.** Superabsorbant selon la revendication 1, **caractérisé en ce que** M représente un ion de métal alcalin ou un équivalent d'un ion de métal alcalino-terreux ou de zinc ; $R^1$ représente un groupe hydroxy ou amino ; $R^2$ représente H ou alkyle, et $R^3$ représente COOM ou $COOR^4$, lorsque $R^3$ représente COOM, M dans ce radical COOM représentant H, un ion de métal alcalin ou un équivalent d'un ion de métal alcalino-terreux et, lorsque $R^3$ représente $COOR^4$, $R^4$ représentant alkyle en $C_1$-$C_6$.

**3.** Superabsorbant selon la revendication 1, **caractérisé en ce que** M représente un ion de métal alcalin ou un équivalent d'un ion de métal alcalino-terreux ou de zinc ; $R^1$ représente un groupe hydroxy ou amino ; $R^2$ représente aryle, qui est éventuellement substitué ; et $R^3$ représente un atome d'hydrogène.

**4.** Superabsorbant selon la revendication 1, **caractérisé en ce qu'**il contient un mélange du sel de sodium de l'acide 2-hydroxy-2-sulfinato-acétique, du sel de disodium de l'acide 2-hydroxy-2-sulfonato-acétique et de bisulfite de sodium.

**5.** Superabsorbant selon l'une quelconque des revendications 1 à 4, **caractérisé en ce qu'**il contient au moins 0,0001 % en poids et au plus 3 % en poids d'un composé de formule (I), ses sels ou des mélanges de tels composés et/ou leurs sels, à chaque fois par rapport au poids total du superabsorbant selon l'invention.

**6.** Procédé de fabrication d'un superabsorbant défini dans les revendications 1 à 5, comprenant le mélange d'un composé de formule (I), de sels de celui-ci ou de mélanges de tels composés et/ou leurs sels avec un superabsorbant et la post-réticulation en surface du superabsorbant, la réaction de post-réticulation étant réalisée à une pression

partielle de gaz oxydants inférieure à 140 mbars.

7. Article pour l'absorption de liquides, contenant un des superabsorbants définis dans les revendications 1 à 5.

8. Procédé de fabrication d'articles pour l'absorption de liquide, **caractérisé en ce qu'**un des superabsorbants définis dans les revendications 1 à 5 est utilisé lors de la fabrication.

9. Superabsorbant, contenant un composé de formule (I)

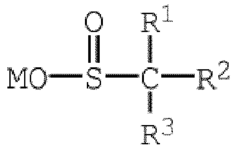

(I),

dans laquelle

M représente un atome d'hydrogène, un ion ammonium, un ion de métal monovalent ou un équivalent d'un ion de métal bivalent des groupes 1, 2, 8, 9, 10, 12 ou 14 du tableau périodique des éléments ;
$R^1$ représente OH ou $NR^4R^5$, $R^4$ et $R^5$ représentant indépendamment l'un de l'autre H ou alkyle en $C_1$-$C_6$ ;
$R^2$ représente H ou un groupe alkyle, alcényle, cycloalkyle ou aryle, ce groupe comprenant éventuellement 1, 2 ou 3 substituants, qui sont choisis indépendamment les uns des autres parmi alkyle en $C_1$-$C_6$, OH, O-alkyle en $C_1$-$C_6$, halogène et $CF_3$ ; et
$R^3$ représente COOM, $SO_3M$, $COR^4$, $CONR^4R^5$ ou $COOR^4$, M, $R^4$ et $R^5$ ayant les significations indiquées précédemment ou, lorsque $R^2$ représente aryle, qui est éventuellement substitué tel qu'indiqué précédemment, représente également H,
ses sels ou des mélanges de tels composés et/ou leurs sels, **caractérisé en ce qu'**il contient en outre un phosphinate et/ou un phosphonate.

10. Superabsorbant selon la revendication 9, **caractérisé en ce que** M représente un ion de métal alcalin ou un équivalent d'un ion de métal alcalino-terreux ou de zinc ; $R^1$ représente un groupe hydroxy ou amino ; $R^2$ représente H ou alkyle, et $R^3$ représente COOM ou $COOR^4$, lorsque $R^3$ représente COOM, M dans ce radical COOM représentant H, un ion de métal alcalin ou un équivalent d'un ion de métal alcalino-terreux et, lorsque $R^3$ représente $COOR^4$, $R^4$ représentant alkyle en $C_1$-$C_6$.

11. Superabsorbant selon la revendication 9, **caractérisé en ce que** M représente un ion de métal alcalin ou un équivalent d'un ion de métal alcalino-terreux ou de zinc ; $R^1$ représente un groupe hydroxy ou amino ; $R^2$ représente aryle, qui est éventuellement substitué ; et $R^3$ représente un atome d'hydrogène.

12. Superabsorbant selon la revendication 9, **caractérisé en ce qu'**il contient un mélange du sel de sodium de l'acide 2-hydroxy-2-sulfinato-acétique, du sel de disodium de l'acide 2-hydroxy-2-sulfonato-acétique et de bisulfite de sodium.

13. Superabsorbant selon l'une quelconque des revendications 9 à 12, **caractérisé en ce qu'**il contient au moins 0,0001 % en poids et au plus 3 % en poids d'un composé de formule (I), ses sels ou des mélanges de tels composés et/ou leurs sels, à chaque fois par rapport au poids total du superabsorbant selon l'invention.

14. Superabsorbant selon l'une quelconque des revendications 9 à 13, **caractérisé en ce qu'**il est post-réticulé en surface.

15. Procédé de fabrication d'un superabsorbant défini dans les revendications 9 à 14, comprenant le mélange d'un composé de formule (I), de sels de celui-ci ou de mélanges de tels composés et/ou leurs sels, ainsi que d'un phosphinate et/ou d'un phosphonate avec un superabsorbant.

16. Article pour l'absorption de liquides, contenant un des superabsorbants définis dans les revendications 9 à 14.

17. Procédé de fabrication d'articles pour l'absorption de liquide, **caractérisé en ce qu'**un des superabsorbants définis

dans les revendications 9 à 14 est utilisé lors de la fabrication.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008055856 A1
- JP 5086251 A
- WO 03059962 A1
- US 20050085604 A1
- WO 03014172 A2
- WO 0055245 A1
- WO 2006058682 A
- EP 505163 A1
- EP 668080 A2
- EP 1570869 A1
- EP 386897 A2
- EP 441975 A1
- EP 605215 A1
- EP 1645596 A1
- EP 1577349 A1
- US 20090023848 A1
- EP 1199315 A2
- WO 9918067 A1
- WO 2004084962 A1
- EP 2008051009 W
- EP 2008051010 W
- WO 2002055469 A1
- WO 2003078378 A1
- WO 2004035514 A1
- EP 530438 A1
- EP 547847 A1
- EP 559476 A1
- EP 632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 200232962 A2
- WO 200138402 A1
- DE 3825366 A1
- US 6241928 B
- EP 0534228 A1
- EP 1191051 A2
- US 20030181115 A
- US 20040019342 A
- EP 0377199 A2
- EP 0445641 A1
- US 5026806 A
- EP 0655465 A1
- EP 0377191 A2
- EP 0640330 A1


### In der Beschreibung aufgeführte Nicht-Patentliteratur


- Modern Superabsorbent Polymer Technology. J. Wiley & Sons, New York, U.S.A. / Wiley-VCH, Weinheim, Germany, 1997
- **THOMAS RICHTER.** Zerstäuben von Flüssigkeiten. Expert-Verlag, 2004, vol. 660
- Standard Test Methods for the Nonwovens Industry. 2005